# EUROPEAN PATENT APPLICATION

(11) **EP 2 690 438 A1**
(43) Date of publication of application: **29.01.2014**
(21) Application number: 12177602.5
(22) Date of filing: 24.07.2012
(51) Int. Cl.: G01N 33/569, A61K 31/702, A61K 39/002, C07H 3/06

(54) **Synthesis of diverse glycosylphosphatidylinositol glycans and glycolipids from toxoplasma gondii and their application as diagnostic markers and vaccines**

(71) Applicant: Max-Planck-Gesellschaft zur Förderung der Wissenschaften e.V., 80539 München (DE)
(72) Inventor: Azzouz, Nahid, 10711 Berlin (DE); Götze, Sebastian, Berlin 12163 (DE); Seeberger, Peter H., 14532 Klein Machnow (DE); Silva, Daniel Varon, 14165 Berlin (DE); Tsai, Yu-Hsuan, 12207 Berlin (DE)
(74) Representative: Arth, Hans-Lothar

(57) **Abstract**

The present invention relates to the synthesis of compounds derived from glycosylphosphatidylinositol (GPI) glycolipids from *Toxoplasma gondii* and the resulting products obtained. These synthetic compounds are suitable for diagnosis of toxoplasmosis and for use as a vaccine against diseases caused by infection with *T. gondii.*

## Description

The present invention relates to the synthesis of GPI-related surface antigens of the parasite *Toxoplasma gondii* (*T. gondii*) and the resulting products obtained. These synthetic compounds are suitable for diagnosis of toxoplasmosis as well as a vaccine against toxoplasmosis, a diseases caused by infection with *T. gondii.*

Glycosylphosphatidylinositols (GPIs) are complex glycolipids that are found in eukaryotic cells either attached to the C-terminus of proteins or in free form. These complex glycolipids feature a phosphoethanolamine unit connecting the C-terminus of the protein to the glycan, a conserved pseudopentasaccharide core of H₂N(CH₂)₂OPO₃H6Manα1→2Manα1→6Manα1→4GlcNα1→6*myo*-Ino1-OPO₃H and a lipid attached to the core glycan via a phosphodiester linkage. The conserved GPI structure can be further decorated by various substituents including additional phosphoethanolamine units, an additional fatty acid ester at C2 position of *myo*inositol and oligosaccharide branch at C3 or C4 of Manl. The constitutive identity of the lipid subunit is variable and may include diacylglycerol, alkylacylglycerol or a ceramide, with chains of different length and varying degrees of unsaturation. When GPIs are isolated from natural sources they are often obtained as heterogeneous mixtures especially in respect to the glycan and lipid subunit. GPIs isolated from different species and, in some cases, from different tissues of the same organism, feature significant structural differences. The primary biological role of GPIs is to localize the attached protein to the outer surface of the plasma membrane bilayer. It is suggested that GPIs are responsible for the association of anchored proteins with lipid rafts and are, thereby, involved in diverse processes such as regulation of innate immunity, protein trafficking, and antigen presentation. Due to heterogeneity of GPIs isolated from biological samples and their amphiphilic character, which renders purification of GPI structures challenging; homogeneous samples of these glycolipids are only accessible via chemical synthesis.

Toxoplasmosis is a parasitic disease caused by the protozoa *T. gondii. T. gondii* is ubiquitous in birds and mammals but the primary host is the family of Felidae (like domestic cats). A third to a half of the human population will have a toxoplasmosis infection at some point in their lives but very few have symptoms. During the first few weeks after exposure, the infection typically causes no symptoms or a mild, flu-like illness: swollen lymph nodes, high temperature or muscle aches. However in most immunocompetent patients, the infection enters a latent phase, during which only bradyzoites are present, forming cysts in nervous and muscle tissues. Thereafter, the parasite rarely causes any symptoms in otherwise healthy adults. Along with immunosuppression reactivation of a latent infection may occur and manifests primarily as toxoplasmic encephalitis. Therefore, anyone with a compromised immune system is at risk for serious problems from toxoplasmosis. These individuals include those undergoing chemotherapy, people suffering from HIV/AIDS or other immune disorders and organ-transplant recipients. The parasite can cause encephalitis (inflammation of the brain) and neurologic diseases, and can affect the heart, liver, inner ears, and eyes. Congenital toxoplasmosis is also more serious and occurs when a woman becomes infected during pregnancy and passes the infection on to her fetus. Women infected before conception normally do not transmit toxoplasmosis to the fetus unless the infection is reactivated during pregnancy by immunosuppression. Disease in neonates may be severe, particularly if acquired early in pregnancy. Even spontaneous abortion and stillbirth may occur. Other symptoms that may occur are: low birth weight, fever, jaundice, abnormalities of the retina, mental retardation, abnormal head size, convulsions, and brain calcification.

Universal screening of pregnant women for example is cost saving at an expected cost of $390 per child screened, inclusive of societal cost of remaining developmental disabilities, compared to an expected societal cost of congenital toxoplasmosis of $1010 per birth under the "no maternal screening" alternative. Thus, cost savings of $620 per child are predicted would this universal maternal screening program be introduced in the US. With an estimated 4 million births per year nearly $2.5 billion could be saved annually compared to no maternal screening (Stillwaggon et al. PLoS Negl Trop Dis. 2011; 5(9), e1333). The diagnosis of toxoplasmosis can be done using a variety of methods. The difficulty lies in the length of time which is needed and in determining whether the infection is acute or chronic (latent). Acute infection can best be verified by isolating *T. gondii* parasite or *T. gondii* DNA from the patient's blood or finding tachyzoites in tissue or bodily fluids. Congenital infection of fetuses can be identified by the presence of cysts in the placenta or fetus. Of particular interest is determining acute infection in pregnant women, due to the risk of congenital toxoplasmosis. This is complicated by the fact that many women have existing IgG and IgM antibodies to *T. gondii* from infection in the past. There are effective diagnostic techniques that monitor changes in the mother's antibody expression over time, but quick diagnosis is greatly preferred because fetuses often rapidly become infected. The Robert-Koch-Institute recommends a serological diagnosis using three subsequently steps:
1. Toxoplasma-antibody screening test: The most commonly used serologic tests detect the presence of anti-*T gondii* IgG antibodies. IgG antibodies can be detected with the Sabin-Feldman dye test (considered the gold standard), indirect fluorescent antibody (IFA) or agglutination. If tests applied to specific total antibodies against *T*. *gondii* as well as to IgG antibodies are negative, then there is neither an infection nor immunity. If the test for total antibodies is negative an infection can be ruled out. Because screening tests based on IgG used in the early phase of infection can still be negative, they must be supplemented especially in pregnant women with an IgM test.
2. Toxoplasma IgM antibody test: If such test results are negative (but positive IgG-Ab test) it can be assumed that an inactive (latent) toxoplasma infection exists. Further studies are not required. If the test is positive further evaluation must be done, especially during pregnancy or if differentiated clinical symptoms exists.
3. Toxoplasma fact-finding process: This includes in particular the determination of the avidity of IgG antibodies, the IgA antibody detection, immunoblot and quantitative research methods. Such a further determination using PCR and histological tests is very expensive.

Hence the diagnosis of *T. gondii* infection, especially of acute toxoplasmosis during pregnancy, is still difficult and time consuming. Therefore there is still need for an effective diagnostic test which allows a fast and reliable diagnosis.

From epidemiological view the prevention of infection is the most important. It is essential to prevent a primary infection during pregnancy. For this it is necessary to know whether there is immunity or not. So far the only possibility for prevention is to avoid acquisition of *T. gondii* infection. These persons should avoid contact with materials that may be contaminated with cat feces and the contact with raw meat. The development of a vaccine based on a defined antigen against *T. gondii,* which produces a sterile immunity is therefore of highest interest.

The cell membrane of *T. gondii* contains two GPIs that differ only by the presence of an additional α-glucose in the side chain. While one GPI is used as a membrane anchor for proteins and surface antigens of the parasite, the other GPI is a free glycolipid on to the plasma membrane and is also known as the low molecular weight antigen of *T. gondii* that elicits a specific IgM immune response in humans and during an acute toxoplasmosis. Both GPIs of *T. gondii* stimulate the production of the cytokine TNF-α in macrophages. Hence the GPIs of *T. gondii* seem to be promising candidates for the development of a vaccine and a diagnostic test.

Such uses rely on the availability of a diverse set of homogeneous GPI structures from *T. gondii.* Due to heterogeneity of GPIs isolated from biological samples and their amphiphilic character, which renders purification of GPI structures challenging, homogeneous samples of these glycolipids are only accessible via chemical synthesis. Further literature shows that isolated GPI structures contain also other glycolipids which could cause false positive results. (Dao et al. 2003 Eur. J. Clin. Microbiol. Infect. Dis., 22, 418-421). With this in mind, the inventors initiated a synthetic program to address the need for a diverse set of homogeneous GPIs and their analogues as a basis for vaccines and diagnostic devices.

Therefore the objective of the present invention is to provide a synthesis of compounds derived from GPIs of *T. gondii* where the resulting products are suitable for use in a diagnostic test of toxoplasmosis and for use as a vaccine for humans and animals against diseases caused by infection with *T. gondii.*

Further preferred embodiments of the present invention are disclosed in the dependent claims, the description and the examples.

### Description of the invention

Thus, the present invention relates to a diagnostic device comprising at least one compound of general formula (I) 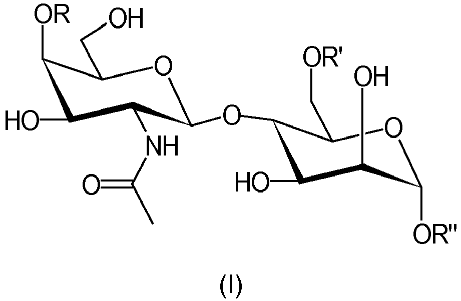 wherein
R represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -Ph, -CH₂-Ph, or 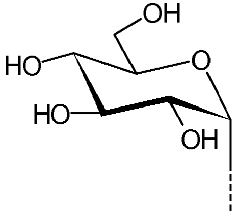
R' represents -H or 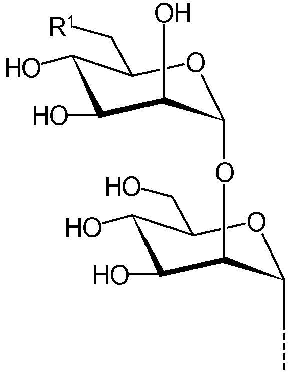
R¹ represents -OH or -OP(O)(OH)-O-X-NH₂;
X represents -CH₂-, -C₂H₄-, -C₃H₆-, -C₄H₈-, -C₅H₁₀- or -C₆H₁₂-,
R" represents -L-R², -(C₂H₄O)ᵣ-CH₂-R², -(C₂H_{4O})ᵣ-C₂H₄-R² or 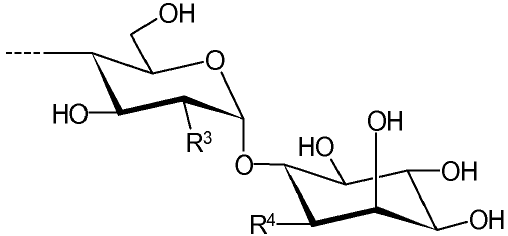
R² and R³ represent independently of each other -H, -OH, -NH₂, -SH, -N₃, -NHCOCH₃, -NHCOCH₂CH₃, or -NHCOCH₂CH₂CH₃;
R⁴ represents -OH, -SO₂(R⁵), -OSO₂(R⁵), -OSO₂(OR⁵), or -OP(O)(OR⁵)(OR⁶);
R⁵ and R⁶ represent independently of each other -H, -L-OH, -(C₂H_{4O})ᵣ-CH₂-OH or -(C₂H₄O)ᵣ-C₂H₄-OH;
L represents a linker;
and r is an integer from 1 to 40,
for diagnosis of toxoplasmosis and wherein the at least one compound of general formula (I) is immobilized on a carrier by covalent bonding.

Another aspect of the present invention refers to the compounds of general formula (I) 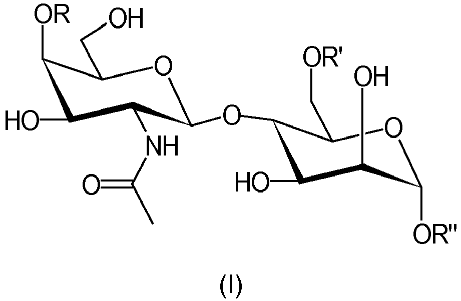 wherein
R represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -Ph, -CH₂-Ph, or 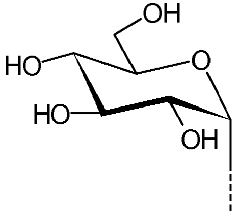
R' represents -H or 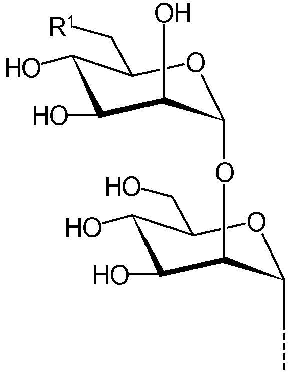
R¹ represents-OH or -OP(O)(OH)-O-C₂H₄-NH₂;
R" represents -L-R² -(C₂H₄O)ᵣ-CH₂-R², -(C²H₄O)ᵣ-C₂H₄-R² or 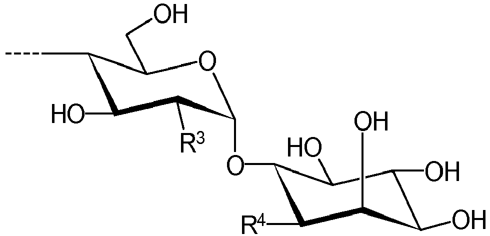
R² and R³ represent independently of each other -H, -OH, -NH₂, -SH, -N₃, -NHCOCH₃, -NHCOCH₂CH₃ or -NHCOCH₂CH₂CH₃;
R⁴ represents -OH, -SO₂(R⁵), -OSO₂(R⁵), -OSO₂(OR⁵), or -OP(O)(OR⁵)(OR⁶);
R⁵ and R⁶ represent independently of each other -H, -L-OH, -(C₂H₄O)ᵣCH₂-OH or -(C₂H₄O)ᵣ-C₂H₄-OH;
L represents a linker;
and r is an integer from 1 to 40.

Preferably one of R⁵ and R⁶ is hydrogen or R⁶ is hydrogen and R⁵ is different from hydrogen.

In above formula (I) L represents any suitable linker. Preferably L represents a linker containing up to 50 carbon atoms. Further preferred is that this linker L is linked through a carbon atom of the linker to the SH group and through the same or preferably another carbon atom of the linker to the -SO₂-, -OSO₂-, -OSO₂-O- or the phosphate group -OP(O)(O-)(O-) in the residues -SO₂-L-OH, -OSO₂-L-OH, -OSO₂-O-L-OH, -OP(O)(OH)(O-L-OH) or -OP(O)(O-L-OH)(O-L-OH). This carbon atom linked linker contains up to 50 carbon atoms and preferably up to 40 carbon atoms and more preferably between 3 and 35 carbon atoms and most preferably between 5 and 30 carbon atoms.

More preferably L represents -L¹-L²-L³-, -L¹-L³-, -L¹-, or -L¹-L²- L⁴-L⁵-L³-, wherein
L¹ and L⁴ represent independently of each other -(CH₂)ₙ-, -(CH₂)ₘ-, -(CH₂)ₙ-CHR⁷-(CH₂)ₘ-, -(CH₂)ₙ-CR⁷R⁸-(CH₂)ₘ-, -O-C₆H₄-, -m-C₆H₄-, -P-C₆H₄-;
L² and L⁵ represent independently of each other -(CH₂)ₚ-, -(CH₂)_{q}-, -CHR⁹-, -CR⁹R¹⁰-, -O-, -S-, -CO-, -COO-, -O-CO-, -NH-CO-, -CO-NH-, -NH-CO-NH-, -_{O}-C₆H₄-, -m-C₆H₄-, -P-C₆H₄-, -NR¹¹-, -CH=CH-; 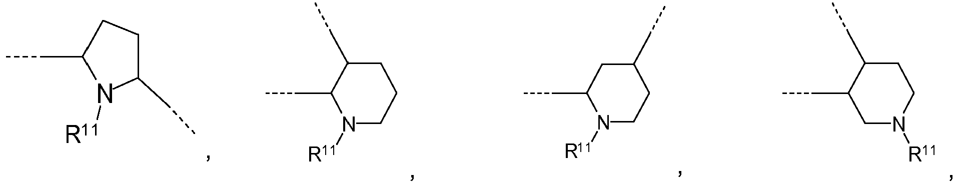 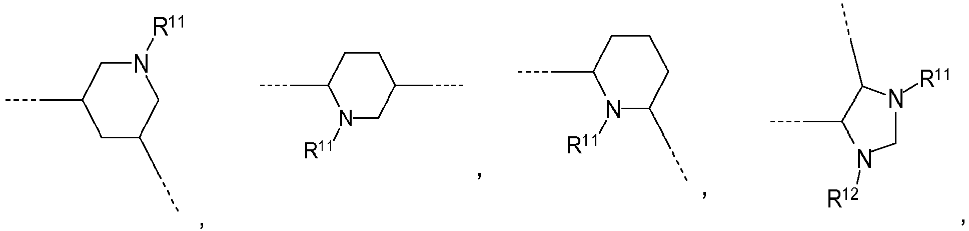 L³ represents -(CH₂)ᵣ-, -(CH₂)ᵣ-CR¹³R¹⁴-(CH₂)ₛ-, -O-C₆H₄-, -m-C₆H₄-, -p-C₆H₄-;
R⁷ to R¹⁰, R¹³ and R¹⁴ represent independently of each other -H, -NH₂, -OH, -OCH₃, -OC₂H₅, -OC₃H₇, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH,
R¹¹ and R¹² represent independently of each other cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁ -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH,
n, m, r and s represent independently of each other an integer from 1 to 20;
p and q represent independently of each other an integer from 0 to 5.

Preferred substituents for R are: -H and

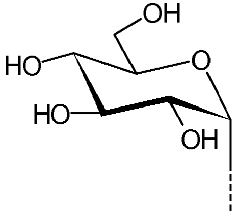

Preferred substituents for R" are: -C₅H₁₀-NH₂, and 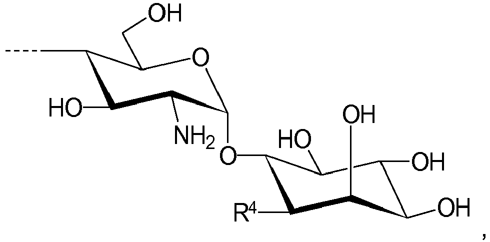 wherein R⁴ has the meaning as defined herein.

Particularly preferred are compounds of the formula (I), wherein R' is:

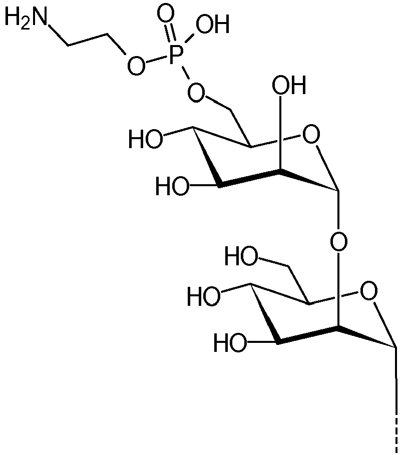

Preferred substituents for R² and R³ are: -NH₂, -SH, -OH, and -NHCOCH₃.

Preferred substituents for R⁵ and R⁶ are: -H, -CH₂-OH, -CH₂-CH₂-OH, -CH₂-CH₂-CH₂-OH, -CH₂-CH₂-CH₂-CH₂-OH, -CH₂-CHOH-CH₂-OH, -CHOH-CH₂-CH₂-OH, -CH-(CH₂-OH)₂, -CH-(CH₂-CH₂-OH)₂, -CH₂-CHOH-CH₂-CH₂-OH, or -CH₂-CH₂-CHOH-CH₂-OH, more preferred are -H, -CH₂-CH₂-OH, and -CH₂-CHOH-CH₂-OH.

Especially preferred are compounds of the formula (II) and (III): 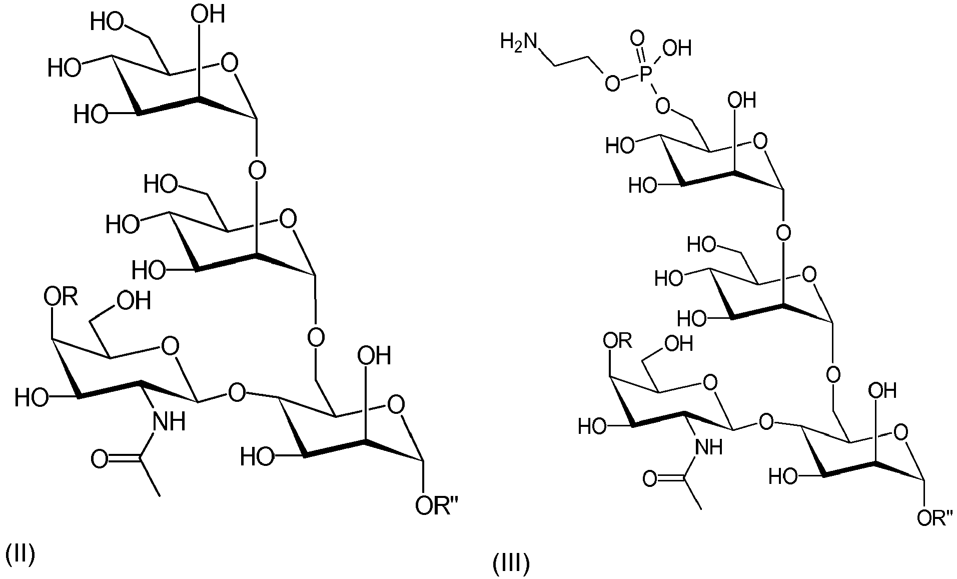 wherein the substituents R' and R" have the meanings as defined herein.

In these formula (II) and (III) **R** represents preferably -H and

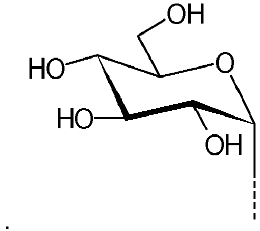

Also in these formula (II) and (III) **R**" -C₅H₁₀-NH₂, and represents preferably

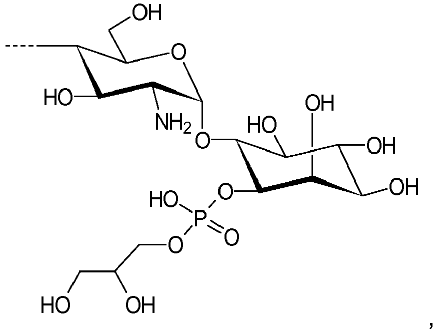

Further preferred are compounds of the formula (IV), (V), (VI) and (VII): 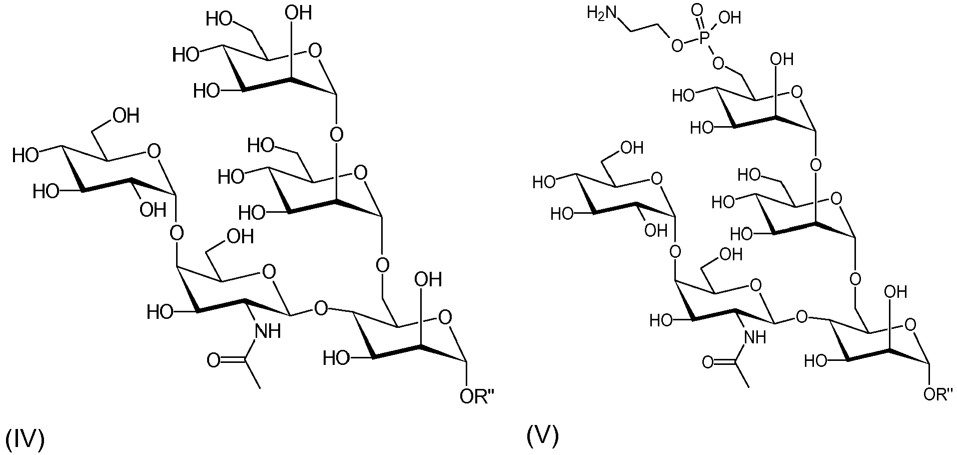 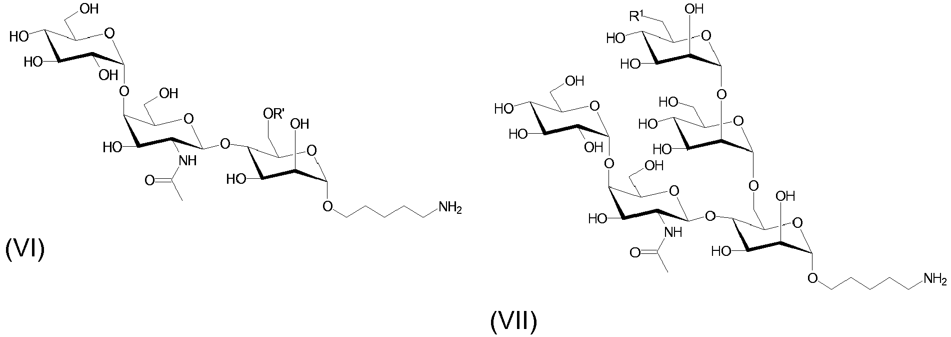 wherein the substituents R', R" and R¹ have the meanings as defined herein.

The compounds falling under general formula (I) - (VII) are novel so that the present invention also relates to compounds of the general formula (I) - (VII) as well as enantiomers, stereoisomeric forms, mixtures of enantiomers, anomers, diastereomers, mixtures of diastereomers, tautomers, hydrates, solvates and racemates and pharmaceutically acceptable salts of these compounds.

In yet another preferred embodiment of the present invention, the compound according to the general formula (I) is selected from the group comprising or consisting of:
1-*O*-5-Aminopentyl α-D-glucopyranosyl-(1→4)-2-deoxy-2-acetamido-β-D-galactopyranosyl-α-D-mannopyranose
1-*O*-5-Aminopentyl α-D-mannopyranosyl-(1→2)-α-D-mannopyranosyl-(1→6)-4-*O*-(α-D-glucopyranosyl-(1→4)-2-deoxy-2-acetamido-β-D-galactopyranosyl)-α-D-mannopyranoside,
1-*O*-5-aminopentyl 6-*O*-(aminoethyl-phosphonato)-α-D-mannopyranosyl-(1→2)-α-D-mannopyranosyl-(1→6)-4-*O*-(α-D-glucopyranosyl-(1→4)-2-deoxy-2-acetamido-β-D-galactopyranosyl)-α-D-mannopyranoside,
6-*O*-(aminoethyl phosphono)-α-D-mannopyranosyl-(1→2)-α-D-mannopyranosyl-(1→6)-4-*O*-(α-D-glucopyranosyl-(1→4)-2-deoxy-2-acetamido-β-D-galactopyranosyl)-α-D-mannopyranosyl-(1→4)-2-amino-2-deoxy-α-D-glucopyranosyl-(1→6)-1-*O*-(2,3-dihydroxypropyl phosphono)-D-*myo*-inositol

Another aspect of the present invention refers to the synthesis of the compounds of the formula (I) 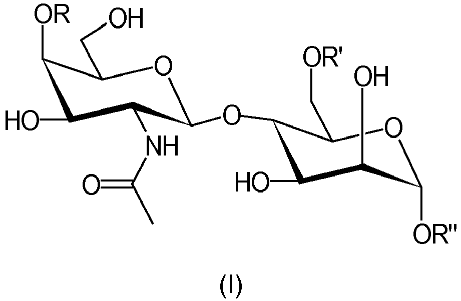 wherein the substituents R, R' and R" have the meanings as defined herein.

Specifically, the compounds of the general formula (I) can be prepared by the general procedure according to following scheme 1: 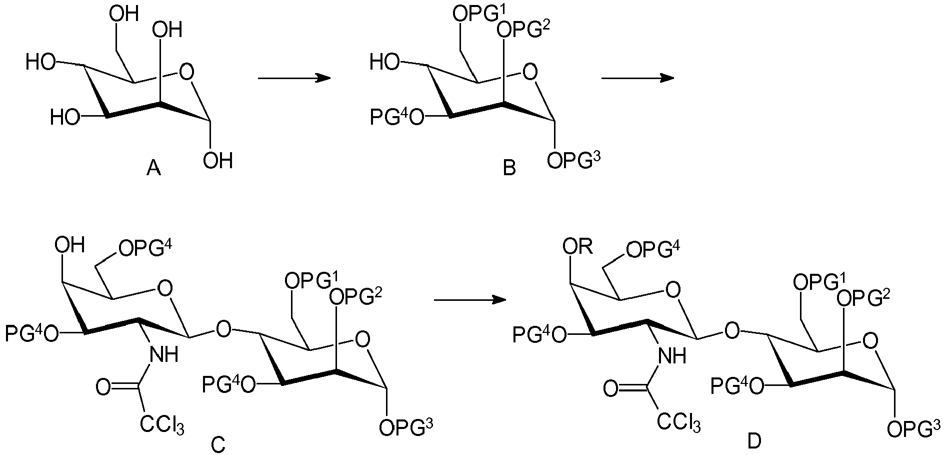 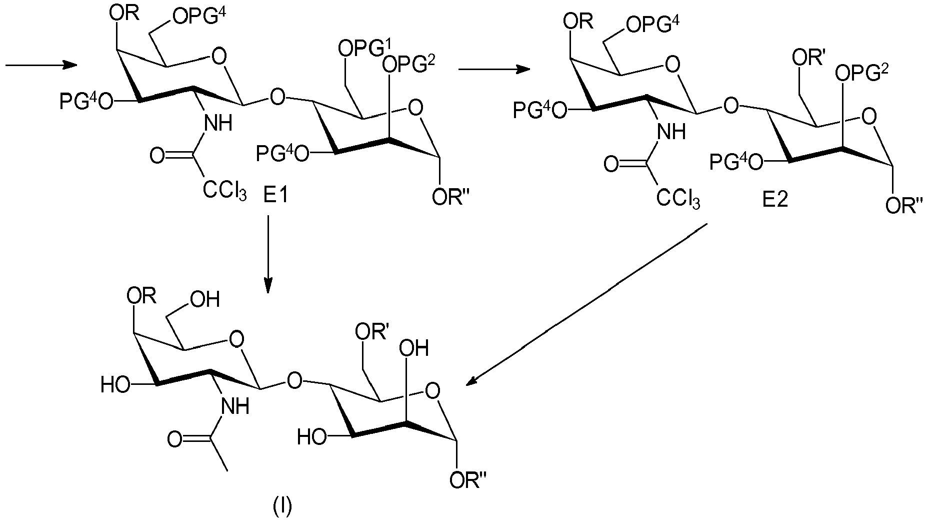 wherein the substituents R, R' and R" have the meanings as defined herein and the protection groups PG¹, PG², PG³ and PG⁴ are commonly used protection groups in organic synthesis, preferably for amines, hydroxyl groups, thiols, imines, carbonyls or other common functional groups.

More specifically, PG¹, PG², PG³ and PG⁴ preferably are suitable protection groups for hydroxyl groups, more preferably different suitable protection groups for hydroxyl groups capable of being removed subsequently one after another by a suitable sequence of deprotection reactions. Preferred protection groups for hydroxyl groups are benzyl, benzoyl, 4-*O*-*p*-methoxybenzyl, allyl, acetyl, methylsulfonylethoxycarbonyl, levulinyl, dimethoxytrityl, 2-naphthylmethyl, triisopropylsilyl, *tert*-butyldimethylsilyl, *tert*-butyldiphenylsilyl, 2-trimethylsilylethoxymethyl. More specifically, in a preferred embodiment of the present invention PG¹ may be *tert*-butyldiphenylsilyl, PG² may be levulinyl, PG³ may be allyl and PG⁴ may be benzyl.

Protection groups for amines may form carbamates such as *tert*-butyloxy carbonyl, 9-fluorenylmethyl carbonyl, allyl carbonyl, trichloroethyl carbonyl, benzylcarboxy carbonyl; carbonyls such as trichloro acetyl or acetyl.

The compounds of the present invention are prepared according to Scheme 1. Mannose **A** is transferred by suitable protection and deprotection strategy for the hydroxyl groups into intermediate compound **B**. A suitable protection strategy for the synthesis of an intermediate **B** is reported in the prior art. Intermediate **B** is henceforth coupled with a suitable precursor, preferably a phosphate precursor, of a galactosyl moiety at the unprotected hydroxyl group of **B** yielding disaccharide **C**. Subsequently, the substituent R is introduced if different from -H, preferably also by phosphate coupling, resulting in intermediate **D**. In case R is supposed to be -H, the hydroxyl group in compound **C** may be protected with a protection group PG⁴ (not shown in scheme 1). Thereafter, intermediate **D** is deprotected from protection group PG³ and the resulting deprotected **D** is activated for coupling with an alcohol. Preferably, the activation of this deprotected **D** occurs on conversion with trichloroacetonitrile, however any other suitable activation reagent may serve as well. This activated compound is preferably reacted with an alcohol R"-OH for introducing the substituent R" yielding intermediate **E**1. Optionally, the introduced substituent R" may be further modified prior to further reaction steps according to Scheme 1. Subsequently, intermediate **E1** can at this stage be directly transformed into compounds of the general formula (I) by preferably simultaneous deprotection from protection groups PG¹, PG⁴ and PG⁵ or can be converted into intermediate **E2** by deprotection from PG¹ and introduction of substituent R'. Introduction of substituent R' preferably is conducted by reacting the PG¹ deprotected **E1** with a suitable procusor of R' being activated by reaction with trichloroacetonitril. Optionally, the introduced substituent R' may be further modified prior to further reaction steps according to Scheme 1. After introduction of substituent R' intermediate **E2** is converted into compounds of the general formula (I) by preferabyl simultaneous deprotection from protection groups PG⁴ and PG⁵.

The protection strategy is directed to protection groups which can be removed in one final step. Therein, it is preferable that on the one hand several kinds of protection groups can be removed and on the other hand several protection groups of one kind can be removed simultaneously. Such strategy facilitates a subsequent introduction of several different kind of substitunets and further one final deprotection step in order to generate the compounds of teh general formula (I). Therein, the key feature of this strategy is the use of a fully orthogonal set of protecting groups that enable regioselective introduction of the required phosphodiesters and the efficient assembly of the building blocks into the GPI glycans.

Following this general strategy for the synthesis a diverse set of GPI glycolipids can conveniently be prepared via modular assembly from common building blocks.

In a preferred embodiment of the present invention it is also possible to first introduce the substituent R' into compound **D** by deprotection of protection group PG¹ and subsequent conversion with a suitable reactive compound of a precursor of R' resulting compound **E3**. After introduction of the substituent R' the substituent R" is introduced into the resulting compound **E3** by deprotection of protecting group PG³ and conversion with a suitable reactive precursor of the substituent R" yielding a compound **E4** which is subsequently entirely deprotected to compounds of the general formula (I): 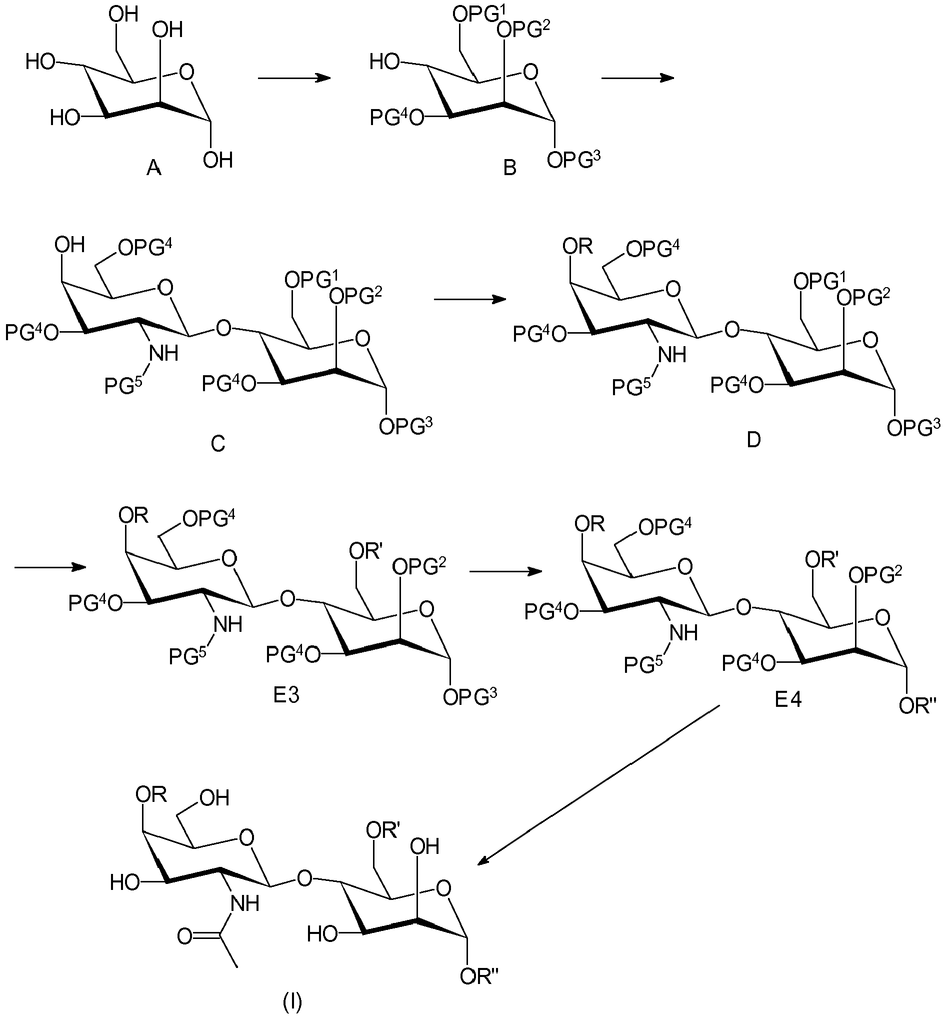 wherein the substituents R, R' and R" have the meanings as defined herein and the protection groups PG¹, PG², PG³, PG⁴ and PG⁵ are commonly used protection groups in organic synthesis, preferably for amines, hydroxyl groups, thiols, imines, carbonyls or other common functional groups.

In case the compounds of the present invention bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Further, it is also possible that the compounds of the present invention bear simultaneously basic and acid groups. Further, it may also occur that these basic and acid groups appear to be in close vicinity to one another enabling an intramolecular proton transfer from the acidic group the basic group. Therefore, in a preferred embodiment of the present invention the compound of the formula (I) may be zwitterionic, bearing at least e.g. one -O- and one -NH₃⁺ group.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The possibility of a synthesis of the compounds according to formula (I) enables production of sufficient and clean amounts of the desired GPI structure which may support research towards understanding of the pathomechanism of *T. gondii* infection and enables designing a vaccine against *T. gondii.*

Surprisingly it was found that the compounds of general formula (I) are also suitable to raise an immune response in an animal and are suitable for vaccination against infectious diseases. Therefore, another aspect of the present invention relates to the use of a compound of general formula (I) for vaccination against toxoplasmosis. One embodiment of the invention is further a compound of the general formula (I) for vaccination against an infection with *T. gondii.* The invention relates also to the use of a compound of general formula (I) for the manufacture of a vaccine against toxoplasmosis.

Further was found that extraordinary potent and stable vaccine can be derived when a compound of general formula (I) is bound to an adjuvant. The present invention relates therefore to a compound of general formula (I) covalently linked to an adjuvant. Particularly preferred is that said adjuvant is a peptidic compound and even more preferred a bacterial peptide or a compound derived from a bacterial peptide.

It is particularly preferred that the adjuvant, which is linked covalently to a compound of general formula (I), is selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid or a mutated tetanus toxoid. The term "toxoid" as used herein refers to a bacterial toxin (usually an exotoxin) whose toxicity has been inactivated or suppressed either by chemical (formalin) or heat treatment, while other properties, typically immunogenicity, are maintained. A mutated toxoid as used herein is a recombinant bacterial toxin which has been amended to be less toxic or even non-toxic by amending the wild-type amino acid sequence. Such a mutation could be a substitution of one or more amino acids. A modified toxoid as used herein is an activated bacterial toxoid a functional group has been introduced or which has been coupled to a linker for binding of any antigen. One possible mutated and modified diphtheria toxoid which may be the non-toxic mutated diphtheria toxin CRM₁₉₇ which is modified by maleimide. CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as meningitis and pneumococcal bacterial infections.

In one aspect of the present invention the compounds of the formula (I) are attached to an adjuvant for the use in a vaccine. The binding to the adjuvant can be accomplished by first providing a suitable adjuvant capable of stimulating the immune system's response to a target antigen, but does not in itself confer immunity as defined above. In a preferred embodiment of the present invention such suitable adjuvant may be CRM₁₉₇. The adjuvant is then functionalized with a chemical substituent which is prone of reacting with a suitable functional group such as hydroxyls, amines or thiols, preferably by formation of a covalent bond. In a preferred embodiment of the present invention the functional group introduced to the adjuvant is prone of reacting with a thiol group. In a preferred embodiment of the present invention the adjuvant is modified by at least functionality of the group comprising or consisting of maleimide; α-iodoacetyl; α-bromoacetyl; *N*-hydroxysuccinimide ester (NHS), 2-pyridyldithiols, thiol and vinyl. The introduction of such functionality to the adjuvant in preferably accomplished by reaction of a suitable adjuvant with a bifunctional linker that bears on one side a functional group prone of reacting with a protein and on the other side one of the above functionalities. In a preferred embodiment of the present invention such a bifunctional linker bears on the on side a vinyl functionality and on the other side a *N*-hydroxysuccinimide ester that is prone of reacting with lysine side amino group. In another preferred embodiment of the present invention such a bifunctional linker bears on the on side a maleimide functionality and on the other side a *N*-hydroxysuccinimide ester that is prone of reacting with lysine side amino group.

For the preferred embodiment wherein a vinyl group is attached to the adjuvant a compound of the formula (I) can be covalently bound to the vinyl group by an thio-en reaction by means of irradiation with UV light and/or in the presence of an initiator upon the formation of a stable thioether bond. For the preferred embodiment wherein a maleimide is attached to the adjuvant the thiol functional group of a compound of the formula (I) adds the double bond of maleimide moiety.

Further, these functionalization reactions are performed in such a way that more than one functional group is bound to the adjuvant. However, it is also possible that just one functional group is put on the adjuvant. In a preferred embodiment of the present invention the number of functional groups bound to the adjuvant after the functionalization reaction ranges preferably from 5 to 100, more preferably from 10 to 50, and most preferably from 10 to 40. After the activation of the adjuvant by one said functionalization reactions the compounds of the present invention are added in order to bind with the thiol functionalization, e.g. to the maleimide double bond by an addition-like reaction. Therein, it is possible to adjust the number of molecule of the formula (I) being added to the adjuvant. The number of added molecules of the formulas (I) can range preferably from 1 to 100, more preferably from 3 to 50, and most preferably from 5 to 15.

Another aspect of the present invention relates to the use of the compound of general formula (I) covalently linked to an adjuvant for vaccination against toxoplasmosis. One embodiment of the invention is further a compound of the general formula (I) covalently linked to an adjuvant for vaccination against an infection with *T. gondii.* The invention relates also to the use of a compound of general formula (I) covalently linked to an adjuvant for the manufacture of a vaccine against toxoplasmosis.

An adjuvant is a pharmacological or immunological agent that modifies the effect of other agents, such as an active agent or vaccine. The term "adjuvant" as used herein refers to a compound used as a carrier protein a compound of general formula (I) is linked to which enhances the recipient's immune response to the compound of general formula (I).

Another aspect of the present invention relates to pharmaceutical formulations and pharmaceutical compositions for vaccination containing a compound of general formula (I) optionally covalently linked to an adjuvant as an active ingredient, together with at least one pharmaceutically acceptable carrier, excipient, solvent and/or diluents.

Further preferred, the pharmaceutical composition is formulated in the form of a lyophilisate or liquid buffer solution.

The compound of general formula (I) optionally covalently linked to an adjuvant can also be administered in form of its pharmaceutically active salt optionally using substantially nontoxic pharmaceutically acceptable carrier, excipients, adjuvants or diluents. The pharmaceutical composition which is used as a vaccine is prepared in a conventional solid or liquid carrier or diluents and may comprise a conventional pharmaceutically-made adjuvant at suitable dosage level in a known way. The inventive pharmaceutical composition may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts. The compounds of the invention of the general formula (I) are present in said vaccine formulation in the range of 10 to 1000 µg/g.

It could be shown that the GPI of *T. gondii* having the additional glucose in the side chain is identical with the known "low molecular weight antigen" of this parasite and IgM antibodies against this structure could be detected in sera from patients with toxoplasmosis (Tomavo et al., 1992, J. Biol Chem 267, 11721-11728). The current ELISA-based diagnostic techniques in order to identify acute toxoplasmosis have a high rate of false-positive results because high IgM responses can be detected even more than a year after a primary infection. Moreover, so far isolates of the parasite are used as antigens for the test, and therefore their quality of production can vary extremely. In literature it has been shown that other glycolipids are included in these isolates which are the reason for the false-positive results (Dao et al. In 2003, J. Clin. Microbiol. Infect. Dis., 22, 418-421). Disadvantages of overtreatment of healthy and infected patients, especially pregnant women, who would require a stressful diagnosis and need medical treatment including pyrimethamine, sulfadiazine, and folinic acid (PSF) after 18 weeks of gestation, which are partially inhibitors of the enzyme dihydrofolate reductase and cause severe side effects such as cardiac arrhythmias and leukopenia, are obvious. Furthermore inhibition of the dihydrofolate reductase can lead to congenital malformations, including neural tube defects, which are of course not very compatible with pregnancy (Stillwaggon et al. PLoS Negl Trop Dis. 2011; 5(9), e1333). A test based on a single synthetic antigen is therefore a clear advantage over the commercially available diagnostic tools.

Moreover the inventors could show that the compounds according to formula (I) can be used in immunological assays for diagnosis of diseases caused by *T. gondii.* Such assays comprise, for instance, microarray and ELISA useful for diagnosis of diseases caused by *T. gondii.* Therefore another aspect of the present invention refers to the use of a compound of formula (I) for diagnosis of toxoplasmosis. Thus especially preferred embodiments of the present invention relate to pure synthetic compounds of formula (I) for diagnosis of toxoplasmosis.

It is preferred that the compound of formula (I) is used for diagnosis of acute toxoplasmosis. Acute toxoplasmosis is characterized by a high-titer of IgM in the serum of the patients and the fact that IgG is not present or only with a low-titer. It is preferred that the compound of formula (I) is used for the differential diagnosis of acute toxoplasmosis, that means that the compound of formula (I) is used in a diagnostic test which allows not only to determine if a patient is infected with *T. gondii* but also to differentiate between an acute infection and a latent or chronic infection.

Thus one especially preferred embodiment of the present invention relates to the use of only one specific defined compound of formula (I) for diagnosis of toxoplasmosis. It is further preferred that the compound of formula (I) used for diagnosis of toxoplasmosis is substantially pure, having a purity of ≥ 95%, preferably ≥ 96%, more preferably ≥ 97%, still more preferably ≥ 98%, and most preferably ≥ 99%. In addition the chemically synthesized compound of formula (I) does not have any microheterogenicity as the oligosaccharides from biological sources do. Nevertheless, the use of a mixture of different compounds of formula (I) for diagnosis of toxoplasmosis is possible but is less preferred.

There are different possibilities for the choice of an assay system in which a compound of formula (I) is used for diagnosis of toxoplasmosis. An assay conducted for diagnostic purposes according to the invention may be an immune assay like a solid-phase enzyme immunoassay (EIA), an enzyme linked immunosorbent assay (ELISA), especially an "indirect" ELISA or a radioimmune assay (RIA). For the use of a compound of formula (I) in such assays it could be necessary to immobilize the compound of formula (I) on a carrier, preferably a solid carrier.

Therefore a compound of formula (I) may be immobilized on a carrier, particularly for diagnostic applications. One preferred embodiment of the present invention is a compound of general formula (I) immobilized on a carrier by covalent bonding. One particularly preferred embodiment of the present invention is a compound of general formula (I) immobilized on a carrier by direct or indirect covalent bonding. Thereby direct covalent bonding is especially preferred.

Further preferred the solid carrier is selected from the group comprising or consisting of: a glass slide, a microtitre plate, test tubes, microspheres, nanoparticle or beads.

It is particularly preferred that the carrier is a glass slide or a microtitre plate. A microtitre plate or microplate or microwell plate, is a flat plate with multiple "wells" used as small test tubes. Typically a microtitre plate having 6, 24, 96, 384 or even 1536 sample wells can be used. Microplates are produced from many different materials, like polycarbonate for microtitre plate used for PCR. The most common is polystyrene as used for most optical detection microplates. It can be colored white by the addition of titanium dioxide for optical absorbance or luminescence detection or black by the addition of carbon for fluorescent biological assays.

"Direct covalent bonding" as used herein refers to immobilization of a compound of general formula (I) by reacting a functional group of the compound of general formula (I) with a functional group of the material the carrier is made from. It is preferred that the functional group of the compound of general formula (I) is R² or R⁴ as defined above. Possible reactive, functional groups of the carrier may be: phenyl, amino groups, hydroxyl groups, thiols, carbonyls, carboxyls, vinyls, halides such as fluorides, chlorides, bromides and iodides, maleimides; succinimide esters.

"Indirect covalent bonding" as used herein refers to immobilization of a compound of general formula (I) on a carrier wherein the compound of general formula (I) is covalently linked to a second compound which mediates the immobilization to the carrier. It is preferred that this second compound is a protein which does not cause an immune reaction. It is important that the second compound itself is most probably not bound by any antibody present in the blood or serum of a patient to avoid false positive results. Further the second compound should be able to be immobilized on the carrier, by covalent or non-covalent bonding. It is preferred that this second compound is selected from the group comprising or consisting of bovine serum albumin (BSA), human serum albumin (HAS), gelatin or casein. The immobilization using indirect covalent bonding therefore refers preferably to covalent bonding of a compound of general formula (I) to a protein as a second compound (eg using the free amino groups of a protein) and subsequently binding of the protein to the carrier by covalent bonding or non covalent interaction between the carrier and the protein. Possible non-covalent interactions are: hydrogen bonds, ionic bonds, van der Waals forces, and hydrophobic interactions. Many polymers, such as polystyrene and polypropylene are hydrophobic in nature. Nevertheless there are also manufacturers which supply carrier having specialized surfaces optimized for different adhesion conditions.

However, immobilization, especially using indirect covalent bonding, may also occur by strong adhesion. Thus, an effective immobilization according to the present invention may be realized not only by chemical bonding, but also unbound by immobilization related to physisorption. As key feature for physisorption acts the phenomenon that the force for adhesion is caused by van der Waals force. The term "unbonded" refers to a bonding other than covalent bonding.

Chemisorption as immobilization form according to the present invention uses chemical bonds between carrier and a compound of formula (I). Such bond may be covalent, but may also be ionic.

In a preferred embodiment of the present invention immobilization of a compound of the formula (I) on a carrier is realized by direct covalent bonding namely a chemical reaction between these two reactants, preferably by a substitution reaction. In a more preferred embodiment of the present invention the carrier is modified with a functional group which is capable of leaving the carrier upon reaction with the compound of the present invention. Such functional group may be bound directly to a composing molecule of the carrier or may be bound to a linker which is directly bound to the composing molecule of the carrier. Thus, in a more preferred embodiment of the present invention the carrier is modified to bearing a suitable leaving group. Suitable leaving groups may be halides such as chlorides, bromides and iodides; hydroxides, oximes, maleimides, succinimide, alcohols and esters. Such leaving group may be or may be incorporated in maleimide; α-iodoacetyl; α-bromoacetyl; N-hydroxysuccinimide ester (NHS) and 2-pyridyldithiols. In yet more preferred embodiment the leaving group on the carrier is capable of reacting with amines, alcohols and thiols, preferably upon proton exchange. In most preferred embodiment of the present invention the carrier is functionalized with a succinimidyl hydroxide functional group, more preferably *N*-succinimidyl hydroxide, which will leave the carrier upon reaction with a compound of the present invention as *N-*hydroxysuccinimide.

Modification of the carrier by introduction of a suitable leaving group is preferably carried out by reaction of an unmodified carrier with a reactive bifunctional molecule, preferably a bifunctional molecule with a molecular bridge or spacer arm between the two functional groups. In a preferred embodiment of the present invention functional groups willingly reacting with the carrier comprise sulfosuccinimide esters and succinimides. One further preferred aspect of the bifunctional linking molecules is the ability of providing the functional group meant to bind with a compound of the formula (I) is an appropriate distance to the carrier. Such an appropriate distance is provided by a molecular bridge or spacer arm of suitable length. Such a molecular bridge or spacer arm may have a length preferably from 3 Å (10⁻¹⁰ m) to 10 nm, more preferably from 5 Å to 50 Å, and most preferably from 6 Å to 30 Å.

Suitable reactive bifunctional molecules for modification of the carrier comprise N-(γ-maleimidobutyryloxy) sulfosuccinimide ester (sulfo-GMBO), succinimidyl (4-iodoacetyl) aminobenzoate (sulfo-SIAN), succinimidyl-3-(bromoacetamido)propionate (SBAP), disuccinimidyl glutarat (DSG), 2-pyridyldithiol-tetraoxatetradecane-N-hydroxysuccinimide (PEG-4-SPDP).

There are also carriers commercially available made from polymers with reactive functional introduced for covalent bonding. One example are microplates named CovaLink™ NH by Thermo scientific which allow covalent binding through a secondary amine group.

Further it is preferred that the carrier is selected from the group comprising or consisting of: a glass slide, a microtitre plate, test tubes microspheres, nanoparticle or beads.

It is particularly preferred that the carrier is a glass slide or a microtitre plate. A Microtitre plate or microplate or microwell plate, is a flat plate with multiple "wells" used as small test tubes. Typically a microtitre plate having 6, 24, 96, 384 or even 1536 sample wells can be used. Microplates are produced from many different materials, like polycarbonate for microtitre plate used for PCR. The most common is polystyrene as used for most optical detection microplates. It can be colored white by the addition of titanium dioxide for optical absorbance or luminescence detection or black by the addition of carbon for fluorescent biological assays.

Another aspect of the present invention is the use of a compound of general formula (I) immobilized on a carrier by covalent bonding for diagnosis of toxoplasmosis. The diagnosis of acute toxoplasmosis is preferred.

One embodiment of the present invention relates to a kit comprising at least one compound of general formula (I) immobilized on a carrier by covalent bonding or the compound of general formula (I) for immobilization on a carrier.

A kit in molecular biology or in medical diagnostics is a package which includes all necessary ingredients for performing a certain method or singular step. Standard chemicals as present in any standard molecular biology or medical laboratory are normally not included. Nevertheless some of these standard chemicals may be indispensable to carry out the diagnosis or the immobilization properly. It is understood that all ingredients are provided in quantities that allow for a proper execution of the desired reactions for the majority of scientific, diagnostic and industrial applications.

Often, but not always, these ingredients are provided in already prepared solutions ready- or close to ready-for-use. There may be also combinations of different ingredients already added together. A further advantage is that such kits use to be verified. Therefore the operator doesn't have to prove again the viability of the diagnostic method and can save on at least some control experiments. Therefore kits are a very popular tool in laboratories in research, diagnostics and industry.

Such a kit according to the invention shall include at least the following components:
A) compound of general formula (I) immobilized on a carrier by covalent bonding
B) at least one antibody, like detection antibody
C) a standard solution

The following components may also be included in such kits:
D) blocking solution
E) wash solution
F) sample buffer

An antibody in the kit may be a specific antibody which can be used as a capture antibody. But preferably it is at least an enzyme-linked secondary antibody used as detection antibody that binds specifically to antibody's Fc region. For quantitative determinations, the optical density (OD) or fluorescence of the sample is compared to a standard curve, which is typically a serial dilution of a known-concentration solution of the target molecule (a standard solution). A blocking solution may be a solution of a non-reacting protein, such as bovine serum albumin or casein, which is added to block any plastic surface in the well that remains uncoated by the antigen. Washing solutions are used to remove unbound components. A sample buffer may be used to dilute the sample of the patient (blood, serum, urine) so that the concentration of the target molecule is in the range which can normally be detected by the test system used.

If the kit shall be allow for the immobilization of a compound of general formula (I) on a solid carrier the kit should include at least:
A) A compound of general formula (I)
B) A carrier, like a microtiter plate

Thereby the carrier may be modified, for example the carrier may be functionalized with a linker molecule as described above.

The following components may also be included in such kits:
C) blocking solution
D) wash solution
E) reaction buffer

### Figures

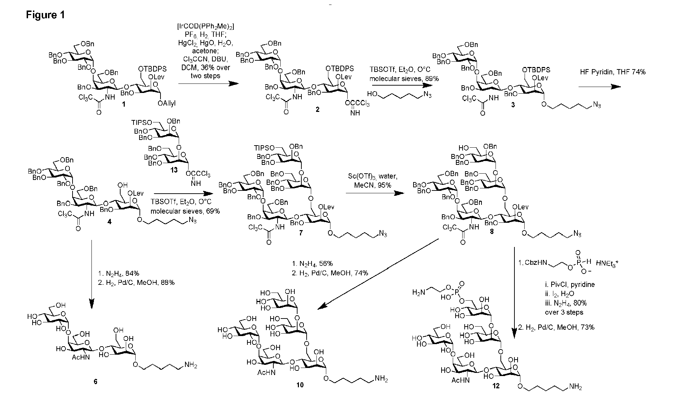

- Figure 1: Synthetic procedure for the glucopyranose substituted compounds 1 to 12.

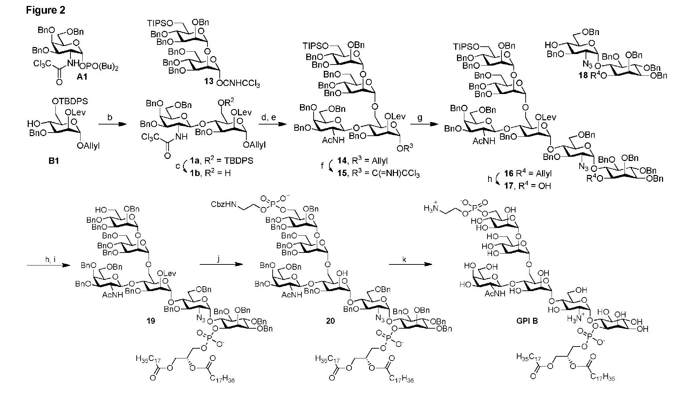

- Figure 2: Synthetic procedure for GPI B.

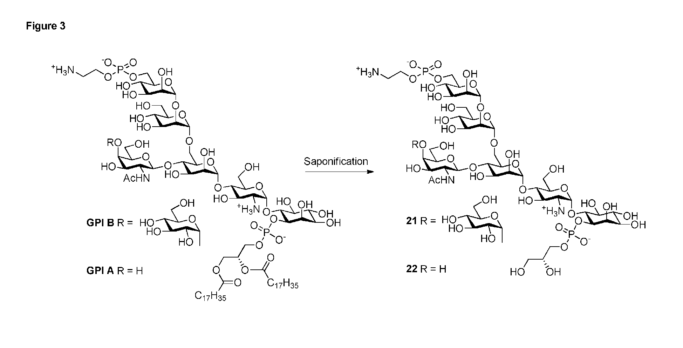

- Figure 3: Synthetic procedure for the ester saponification reaction yielding compounds 21 and 22.
Reagents and conditions: (b) **A1**, TMSOTf, CH₂Cl₂, 4Å MS, -40 °C, 82%; (c) HF•Py, THF, 80%; (d) **13**, TBSOTf, Et₂O, MS 4Å, 0 °C, 74%; (e) Zn, AcOH, 55 °C, 74% (f) i. [Ir(COD)(PPh₂Me)₂]PF₆, H₂, THF; ii. HgCl₂, HgO, H₂O, acetone 69% (two steps); iii. Cl₃CCN, DBU, CH₂Cl₂, 89%; (g) **18**, TBSOTf, Et₂O, MS 4Å, 0 °C, 81 %; (h) i. [Ir(COD)(PPh₂Me)₂]PF₆, H₂, THF; ii. HgCl₂, HgO, H₂O, acetone, 82% (two steps); (i) i. triethylammonium (*S*)-2,3-bis(stearoyloxy)propyl phosphonate, PivCl, Py; ii. I₂, H₂O, 91% (two steps); iii. Sc(OTf)₃, MeCN, CHCl₃, 71%; (j) i. triethylammonium 2-[(*N-*benzyloxycarbonyl)amino]ethyl H-phosphonate, PivCl, Py; ii. I₂, H₂O; iii. H₂NNH₂, AcOH, pyridine, CHCl₃, 80% (three steps); (k) H₂, Pd/C, CHCl₃/MeOH/AcOH/H₂O, 89%.

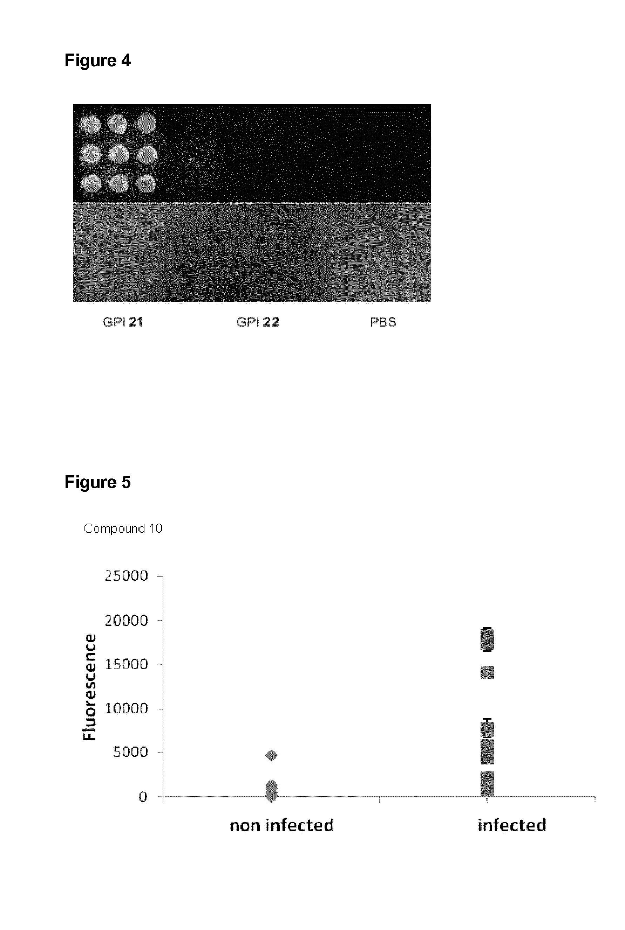

- Figure 4: Microarray after serum analysis. GPI 21 and GPI 22 were printed in a 3x3 pattern together with PBS as blank test. Results for patient with acute toxoplasmosis depicted above, results for patient without toxoplasmosis depicted below.
- Figure 5: Fluorescence measurement of serum for IgG antibodies of taxoplasmosis negative and taxoplasmosis positive patients against compound 10 (10 samples of infected and 10 samples of non infected persons).

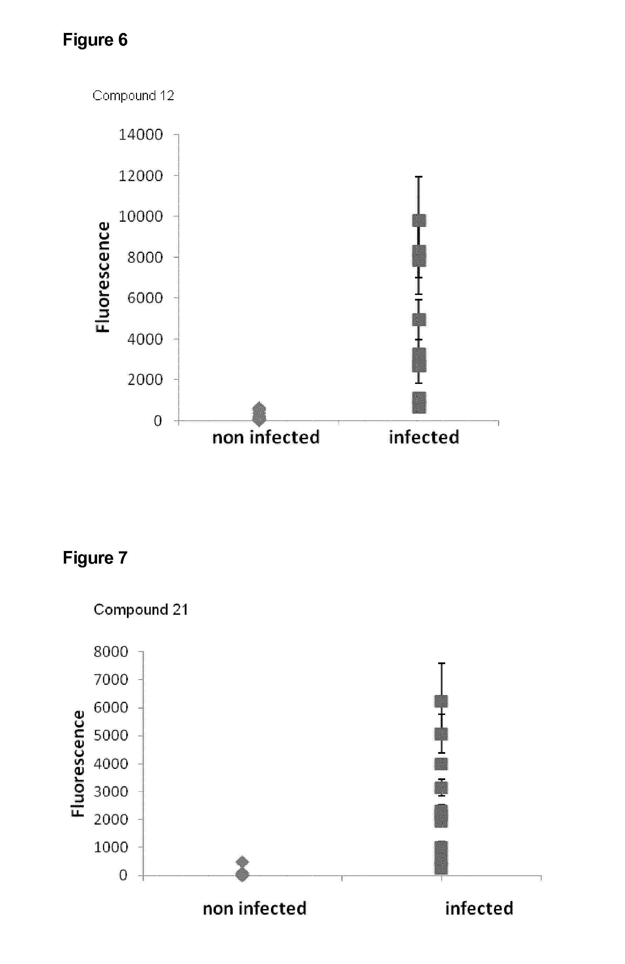

- Figure 6: Fluorescence measurement of serum for IgG antibodies of taxoplasmosis negative and taxoplasmosis positive patients against compound 12 (10 samples of infected and 10 samples of non infected persons).
- Figure 7: Fluorescence measurement of serum for IgG antibodies of taxoplasmosis negative and taxoplasmosis positive patients against compound 21 (10 samples of infected and 10 samples of non infected persons).

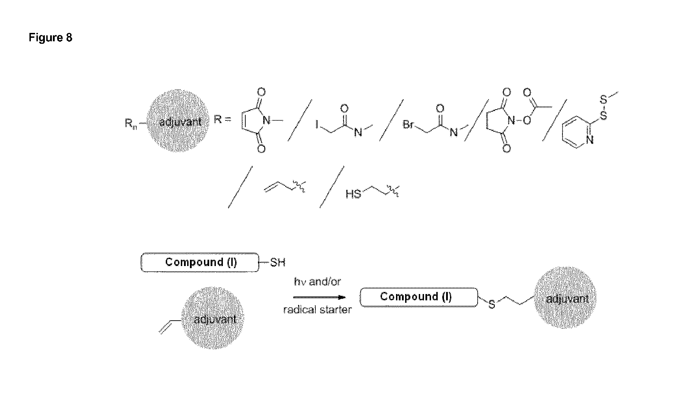

- Figure 8: Possible functional groups being attached to a suitable adjuvant and one possible reaction pathway of attaching a compound of the general formula (I) to a modified adjuvant by an thio-en reaction upon activation by irradiation of light and/or by a radical starter.

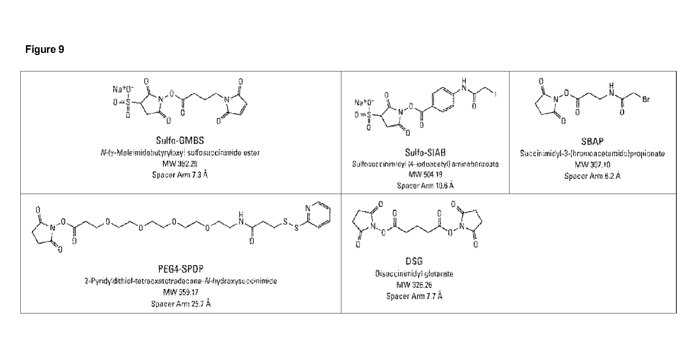

- Figure 9: Selection of compounds suitable for modifying a carrier for subsequent introduction of a compound of the formula (I) to the carrier by direct bonding.

### Examples

Glycosylphosphatidylinositol Glycolipids GPI A was prepared according to literature procedures: GPI A (Tsai et al. 2011, Angew. Chem. Int. Ed. 50, 9961 -9964).

### A Preparation of Examples with R = Glucopyranose

Compound 1 and compound 13 according to the reaction scheme above were prepared to the procedure published in Angewandte Chemie International Edition, 2011, 50, 9961.

### Example 1

### 2,3,4,6-tetra-O-benzyl-α-D-glucopyranosyl-(1→4)-3,6-di-O-benzy)-2-deoxy-2-trichlor-acetamido-β-D-galactopyranosyl-3-O-benzyl-6-O-tertbutyldiphenylsilyl-2-O-levulinyl-α-D-mannopyranosyl trichloroacetimidate

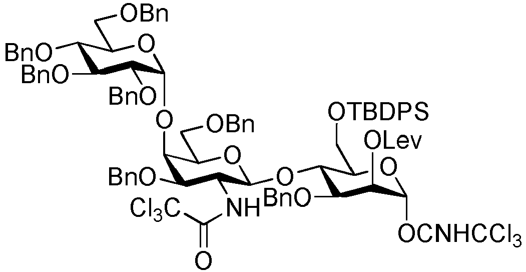

A solution of [IrCOD(PPh₂Me)₂]PF₆ (4.9 mg, 5.8 µmol) in THF (3 mL) was stirred under hydrogen at 20 °C until the colour turned from red to colourless to pale yellow. The hydrogen atmosphere was ex-changed with nitrogen. This solution was then added into a flask with allyl 2,3,4,6-tetra-O-benzyl-α-D-glucopyranosyl-(1→4)-3,6-di-O-benzyl-2-deoxy-2-trichlor-acetamido-β-D-galactopyranosyl-3-O-benzyl-6-O-tertbutyldiphenylsilyl-2-O-levulinyl-α-D-mannopyranoside 1 (300 mg, 0.181 mmol). After overnight stirring, the solvent was removed and the residue was dissolved in a mixture of acetone (3.6 mL) and water (0.4 mL). Mercury(II) chloride (295 mg, 1.09 mmol) and mercury(II) oxide (3.9 mg, 0.018 mmol) were added. After 1 h, the reaction mixture was diluted with chloroform and washed with saturated NaHCO₃(aq), dried over Na₂SO₄, filtered and concentrated to give the crude lactol: R_{f} (SiO₂, EtOAc/Hexane 1:4 = 0.09), which was purified by flash column chromatography to yield an inseparable mixture of anomers. The lactol was dissolved in DCM (1 mL) and 2,2,2-trichloroacetonitrile (0.23 mL, 2.32 mmol) and DBU (1 drop) were added. After 1 h, the solvent was removed to give a brown oil that was purified by flash column chromatography to give imidate 2 (115 mg, 0.065 mmol, 36% yield) as a white foam: R_{f} (SiO₂, EtOAc/Hexane 1:3) = 0.33; [α]^{D}₂₀: + 48.9 (c = 0.35, CHCl₃); ATR-FTIR (cm⁻¹): 3031, 2929, 1719, 1454, 1428, 1361, 1150, 1103, 1028; ¹H NMR (400 MHz, CHCl₃) δ 8.65 (s, 1 H, NH of imidate), 7.77 - 7.68 (m, 4H), 7.45 - 7.14 (m, 35H), 7.12 (dd, J = 6.9, 2.7 Hz, 2H), 7.09 - 7.04 (m, 2H), 6.49 (d, J = 7.5 Hz, 1 H, NH), 6.28 (d, J = 2.0 Hz, 1 H, Man-1), 5.35 (dd, J = 3.3, 2.2 Hz, 1 H, Man-2), 5.13 (d, J = 3.4 Hz, 1 H, Glc-1), 5.07 (d, J = 8.1 Hz, 1 H, GaINAc-1), 4.86 - 4.72 (m, 6H), 4.69 (d, J = 11.2 Hz, 1 H), 4.61 (d, J = 11.0 Hz, 1 H), 4.49 (m, 3H), 4.41 - 4.33 (m, 2H), 4.29 (d, J = 12.0 Hz, 1 H), 4.24 - 4.14 (m, 3H), 4.11 (d, J = 12.2 Hz, 1 H), 4.06 (d, J = 9.4 Hz, 1 H), 3.95 (m, 5H), 3.85 - 3.78 (m, 1 H), 3.78 - 3.71 (m, 1 H), 3.59 (dd, J = 9.9, 3.4 Hz, 1 H), 3.56-3.50 (m, 2H), 3.42 (dd, J = 10.7, 2.0 Hz, 1 H), 3.05 (dd, J = 10.6, 1.6 Hz, 1 H), 2.73 - 2.46 (m, 4H, CH₂ of Lev), 2.08 (s, 3H, Me of Lev), 1.10 (s, 9H, *tert*Bu); ¹³C NMR (101 MHz, CHCl₃) δ 206.09 (ketone of Lev), 171.90, 161.61, 159.83, 138.80, 138.49, 138.39, 138.34, 138.31, 138.00, 137.96, 136.02, 135.78, 133.77, 133.03, 129.91, 128.51, 128.47, 128.43, 128.32, 128.29, 128.28, 128.18, 128.15, 128.00, 127.87, 127.82, 127.74, 127.69, 127.65, 127.59, 127.57, 127.38, 127.32, 100.21 (Glc-1, *J_{C,H}* = 172 Hz), 98.66 (GalNAc-1, *J_{C,H}* = 164 Hz), 95.00 (Man-1, *J_{C,H}* = 181 Hz), 92.45, 90.92 (2xCCl₃), 82.20, 80.10, 77.81, 76.51, 75.31, 75.03, 74.97, 73.88, 73.73, 73.66, 73.53, 73.32, 73.16, 72.41, 71.92, 71.60, 70.88, 68.80, 67.70, 67.45, 62.70, 56.32, 37.96, 29.80, 28.10, 26.81, 19.50.; ESI-MS: m/z [M+Na]⁺ cald 1783.4590 for C₉₂H₉₈Cl₆N₂O₁₈Si, obsd 1783.4538.

### Example 2

### 1-O-5-Azidopentyl 2,3,4,6-tetra-O-benzyl-α-D-glucopyranosyl-(1→4)-3,6-di-O-benzyl-2-deoxy-2-trichlor-acetamido-β-D-galactopyranosyl-3-O-benzyl-6-O-tertbutyldiphenylsilyl-2-O-levulinyl-α-D-mannopyranose

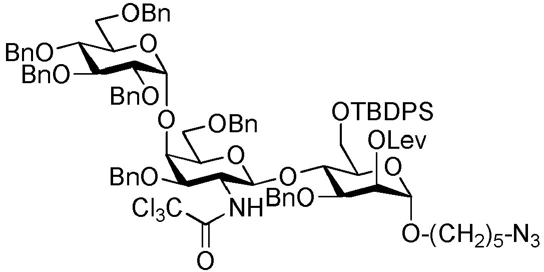

The imidate of Example 1 (115 mg, 0.065mmol) and 5-azidopentan-1-ol (12.7 mg, 0.098mmol) were co evaporated with toluene (3x2 mL) and placed under high vacuum for 30 min. The residue was dissolved in diethylether (2 mL) and powdered molecular sieves (100 mg, 4A) was added. The slurry was stirred for 10 min at r.t. before it was cooled down to 0°C and TBSOTf (15.0 µL, 0.065 mmol) was added. After 1 h at 0°C the reaction was quenched with TEA (50 µL), filtered and evaporated to dryness. The residue was purified by flash column chromatography to give trisaccharide 3 (100 mg, 0.058 mmol, 89%) as a white foam: R_{f} (SiO₂, EtOAc/Hexane 1:2) = 0.58; [α]^{D}₂₀: + 58.2 (c = 1.11, THF); ATR-FTIR (cm⁻¹): 3036, 2926, 2864, 2108, 1683, 1454, 1362, 1093, 1071, 1028; ¹H NMR (400 MHz, CHCl₃) δ 7.80 - 7.71 (m, 4H), 7.47 - 7.14 (m, 39H), 7.09 - 7.05 (m, 2H), 6.46 (d, J = 7.2 Hz, 1 H, NH), 5.29 - 5.25 (m, 2H, GalNAc-1, Man-2), 5.12 (d, J = 3.3 Hz, 1 H, Glc-1), 4.86-4.67 (m, 9H), 4.50 - 4.35 (m, 3H), 4.22-3.99 (m, 10H), 3.97 (dd, J = 9.0, 3.4 Hz, 1 H), 3.91 (dd, J = 11.1, 5.6 Hz, 1 H), 3.82 - 3.69 (m, 3H), 3.66 - 3.56 (m, 2H), 3.47 (dd, J = 8.4, 5.5 Hz, 1 H), 3.41 (dt, J = 9.6, 6.6 Hz, 1 H), 3.38 - 3.32 (m, 2H), 3.27 (t, J = 6.9 Hz, 2H), 2.98 (d, J = 9.2 Hz, 1 H), 2.69 - 2.42 (m, 4H, CH₂ of Lev), 2.07 (s, 3H, Me of Lev), 1.67 - 1.56 (m, 4H), 1.47 - 1.37 (m, 2H), 1.09 (s, 9H, *tert*Bu); ¹³C NMR (101 MHz, CHCl₃) δ 206.40 (ketone of Lev), 172.09, 163.46, 161.76, 138.73, 138.71, 138.39, 138.28, 138.15, 137.92, 137.81, 136.03, 135.65, 133.67, 133.20, 129.95, 129.79, 128.39, 128.37, 128.30, 128.26, 128.22, 128.14, 127.99, 127.93, 127.86, 127.77, 127.68, 127.65, 127.63, 127.60, 127.57, 127.53, 127.48, 127.43, 127.23, 127.09, 126.87, 99.97 (Glc-1), 98.80 (GalNAc-1), 97.13 (Man-1), 92.12 (CCl₃), 82.05, 79.97, 77.77, 77.31, 76.11, 75.94, 75.34, 74.97, 73.76, 73.57, 73.37, 73.26, 73.24, 72.99, 72.50, 71.79, 71.28, 70.68, 69.29, 67.55, 67.40, 67.23, 63.81, 56.80, 51.28, 37.93, 29.73, 28.86, 28.66, 28.11, 26.69, 23.41, 19.33; ESI-MS: m/z [M+Na]⁺ cald 1749.6314 for C₉₅H₁₀₇Cl₃N₄O₁₈Sᵢ, obsd 1749.6305.

### Example 3

### 1-O-5-Azidopentyl 2,3,4,6-tetra-O-benzyl-α-D-glucopyranosyl-(1→4)-3,6-di-O-benzyl-2-deoxy-2-trichlor-acetamido-β-D-galactopyranosyl-3-O-benzyl-2-O-levulinyl-α-D-mannopyranose (4)

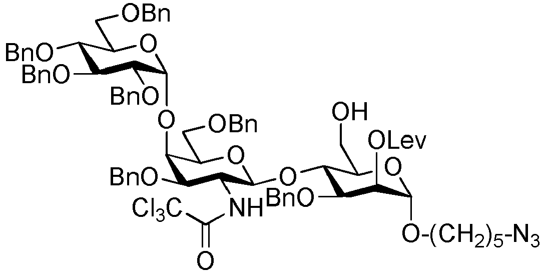

The trisaccharide 3 of Example 2 (100mg, 0.058 mmol) was transferred to a 15 mL Falcon™ tube and dissolved in THF (2 mL). HF.pyridine (200 µL) was added and the solution was stirred for 18 h at r.t. before it was quenched with saturated NaHCO₃(aq), diluted with CHCl₃ (10 mL) dried over Na₂SO₄, filtered and concentrated. The residue was purified by flash column chromatography to give alcohol 4 (66 mg, 0.044 mmol, 77% yield) as a white foam: R_{f} (SiO₂, EtOAc/Hexane 2:1) = 0.20; [α]^{D}₂₀: + 49.2 (c = 0.98, CHCl₃); ATR-FTIR (cm⁻¹): 2927, 2096, 1716, 1522, 1454, 1361, 1070, 1027; ¹H NMR (400 MHz, CHCl₃) δ 7.40 - 7.13 (m, 32H), 7.10 - 7.01 (m, 3H), 5.30 (d, J = 7.9 Hz, 1 H, GalNAc-1), 5.24 (dd, J = 3.2, 1.6 Hz, 1 H, Man-2), 5.08 (d, J = 3.3 Hz, 1 H, Glc-1), 4.87 - 4.61 (m, 9H), 4.44 (dd, J = 16.0, 10.2 Hz, 2H), 4.36 (d, J = 10.6 Hz, 1 H), 4.24 - 4.01 (m, 9H), 3.95 (dd, J = 9.0, 3.3 Hz, 1 H), 3.88 - 3.78 (m, 3H), 3.76 - 3.54 (m, 6H), 3.48 - 3.33 (m, 3H), 3.27 (t, J = 6.9 Hz, 2H), 2.98 (d, J = 9.5 Hz, 1 H), 2.59 - 2.40 (m, 4H, CH₂ of Lev), 2.20 (bs, 1 H, OH), 2.05 (s, 3H, Me of Lev), 1.65 - 1.53 (m, 4H), 1.47 - 1.35 (m, 2H); ¹³C NMR (101 MHz, CHCl₃) δ 206.50 (ketone of Lev), 172.10, 162.03, 138.88, 138.48, 138.43, 138.40, 138.16, 137.93, 128.51, 128.48, 128.46, 128.39, 128.37, 128.32, 128.20, 128.06, 127.82, 127.74, 127.70, 127.65, 127.60, 127.51, 127.42, 127.34, 126.99, 100.15 (Glc-1), 99.00 (GalNAc-1), 97.52 (Man-1), 92.64, 82.10, 80.20, 77.84, 76.22, 76.02, 75.41, 75.07, 73.90, 73.72, 73.63, 73.33, 73.09, 71.84, 71.48, 71.20, 70.83, 69.31 (Man-2), 67.75, 67.59, 61.99, 56.56, 51.34, 37.94, 29.72, 28.95, 28.70, 28.16, 23.45; ESI-MS: m/z [M+Na]⁺ cald 1511.5128 for C₇₉H₈₉Cl₃N₄O₁₈, obsd 1511.5059.

### Example 4

### 1-O-5-Azidopentyl 2,3,4,6-tetra-O-benzyl-α-D-glucopyranosyl-(1→4)-3,6-di-O-benzyl-2-deoxy-2-trichlor-acetamido-β-D-galactopyranosyl-3-O-benzyl-α-D-mannopyranose (5)

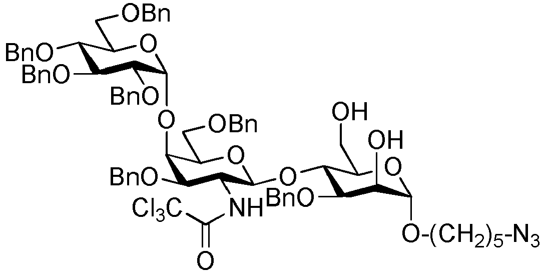

The alcohol 4 of Example 3 (9.6 mg, 6.45 µmol) was dissolved in CHCl₃ (1 mL) and hydrazine monohydrate (2 drops, 64 wt%) was added. The solution was stirred for 1 h before the reaction mixture was evaporated to dryness. The residue was purified by flash column chromatography to give azide 5 (7.5 mg, 5.4 µmol, 84% yield) as colorless oil: R_{f} (SiO₂, EtOAc/Hexane 3:1) = 0.68; [α]^{D}₂₀: + 46.2 (c = 0.75, CHCl₃); ATR-FTIR (cm⁻¹): 2927, 1095, 1716, 1497, 1454, 1060; ¹H NMR (600 MHz, CHCl₃) δ 7.40 - 7.14 (m, 28H), 7.13 - 7.09 (m, 2H), 7.07 (t, J = 7.8 Hz, 1 H), 6.93 (t, J = 7.5 Hz, 2H), 6.84 (d, J = 6.2 Hz, 1 H), 6.78 (d, J = 7.4 Hz, 2H), 5.29 (d, J = 8.0 Hz, 1 H, GaINAc-1), 5.05 (dd, J = 12.6, 6.9 Hz, 2H), 4.97 (d, J = 10.8 Hz, 1 H), 4.85 (d, J = 12.0 Hz, 1 H), 4.80 - 4.71 (m, 4H), 4.49 (d, J = 12.1 Hz, 1 H), 4.46 - 4.38 (m, 3H), 4.34 (dd, J = 17.9, 10.7 Hz, 2H), 4.24 - 4.14 (m, 6H), 4.05 (t, J = 8.6 Hz, 1 H), 4.00 - 3.89 (m, 3H), 3.85 (s, 1 H), 3.81 - 3.74 (m, 3H), 3.73 (dd, J = 9.3, 2.4 Hz, 1 H), 3.68 - 3.55 (m, 4H), 3.46 (dd, J = 10.4, 1.5 Hz, 1 H), 3.40 - 3.34 (m, 1 H), 3.27 (t, J = 6.9 Hz, 2H), 2.96 (dd, J = 10.4, 1.7 Hz, 1 H), 1.66 - 1.53 (m, 4H), 1.45 - 1.36 (m, 2H); ¹³C NMR (151 MHz, CHCl₃) δ 162.11 (C=O of amide), 139.51, 138.39, 138.21, 138.14, 137.91, 137.80, 137.58, 128.99, 128.64, 128.59, 128.54, 128.50, 128.43, 128.41, 128.19, 127.98, 127.88, 127.77, 127.66, 127.57, 127.53, 126.05, 100.61, 99.31, 96.71 (GalNAc-1), 92.48 (CCl₃), 82.06, 80.82, 78.96, 77.74, 75.67, 75.43, 75.37, 74.56, 73.61, 73.47, 73.35, 73.05, 72.16, 71.93, 71.29, 69.99, 68.63, 67.42, 67.22, 66.82, 62.16, 55.53, 51.44, 29.12, 28.81, 23.60; ESI-MS: m/z [M+Na]⁺ cald for C₇₄H₈₃Cl₃N₄O₁₆ 1413.4733, obsd 1413.4678.

### Example 5

### 1-O-5-Aminopentyl α-D-glucopyranosyl-(1→4)-2-deoxy-2-acetamido-β-D-galactopyranosyl-α-D-mannopyranose (6)

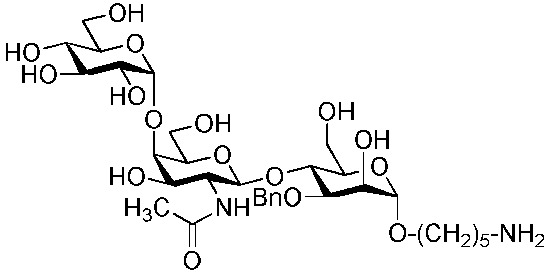

The azide 5 of Example 4 (7.5 mg, 5.4 µmol) was dissolved in MeOH (2 mL) before Pd/C (28.7 mg, 10 wt%) and HOAc (2 drops) were added. Hydrogen was bubbled through this slurry for 10 min before the slurry was stirred for 24 h under the same atmosphere. The slurry was filtered through a syringe filter and concentrated. The residue was purified using a G-25 size exclusion column (running buffer 5% EtOH in water) to yield oligosaccharide 6 (3 mg, 4.8 µmol, 88% yield) as white solid. [α]^{D}₂₀: + 37.9 (c = 0.33, H₂O); ATR-FTIR (cm⁻¹): 3312, 1625, 1316, 1045; ¹H NMR (400 MHz, D₂O) δ 4.96 (d, J = 3.8 Hz, 1 H), 4.88 (s, 1 H), 4.58 (d, J = 8.3 Hz, 1 H), 4.13 (dt, J = 10.3, 2.8 Hz, 1 H), 4.08 - 3.96 (m, 4H), 3.94 - 3.64 (m, 13H), 3.62 - 3.55 (m, 2H), 3.53 - 3.46 (m, 1 H), 3.03 (t, J = 7.6 Hz, 2H), 2.10 (s, 3H), 1.77 - 1.58 (m, 4H), 1.53 - 1.37 (m, 2H); ¹³C NMR (101 MHz, D₂O) δ 160.63 (C=O of amide), 102.14, 100.14, 99.24, 77.80, 76.54, 75.35, 72.63, 71.89, 71.72, 70.96, 70.10, 69.51, 69.48, 69.12, 67.43, 60.42, 60.26, 59.84, 52.36, 39.36, 27.93, 26.59, 22.44 (Me); ESI-MS: m/z [M+Na]⁺ cald for C₂₅H₄₆N₂O₁₆ 631.2920, obsd 631.2854.

### Example 6

### 1-O-5-Azidopentyl 2,3,4-tri-O-benzyl-6-O-triisopropylsilyl-α-D-mannopyranosyl-(1→2)-3,4,6-tri-O-benzyl-α-D-mannopyranosyl-(1→6)-3-O-benzyl-4-O-(2,3,4,6-tetra-O-benzyl-α-D-glucopyranosyl-(1→4)-3,6-di-O-benzyl-2-deoxy-2-trichloracetamido-β-D-galactopyranosyl)-2-O-levulinyl-α-D-mannopyranoside (7)

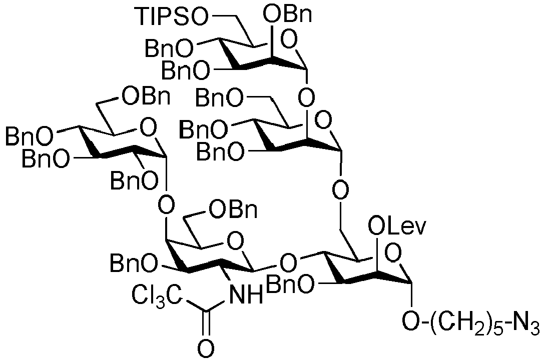

The alcohol 4 of Example 3 (56 mg, 0.038 mmol) and imidate 13 (53mg, 0.045mmol) were co evaporated with toluene (3x2 mL) and placed under high vacuum for 30 min. The residue was dissolved in diethylether (2 mL) and powdered molecular sieve (100 mg, 4A) was added. The slurry was stirred for 10 min at r.t. before it was cooled down to 0°C and TBSOTf (10.4 µL, 0.045 mmol) was added. The reaction mixture was stirred at 0°C for 1 h before it was quenched with TEA (50 µL), diluted with CHCl₃, filtered and concentrated. The residue was purified by flash column chromatography to give pentasaccharide 7 (65 mg, 0.026 mmol, 69% yield) as a white foam: R_{f} (SiO₂, EtOAc/Hexane 1:3) = 0.21; [α]^{D}₂₀: + 38.4 (c = 1.00, CHCl₃); ATR-FTIR (cm⁻¹): 2865, 2097, 1717, 1497, 1454, 1362, 1070; ¹H NMR (400 MHz, CHCl₃) δ 7.37 (d, J = 7.2 Hz, 2H), 7.33 - 7.10 (m, 60H), 7.06 (d, J = 7.4 Hz, 1 H), 7.01 (dd, J = 6.6, 2.9 Hz, 2H), 5.28 (d, J = 1.4 Hz, 1 H), 5.23 (dd, J = 3.4, 1.9 Hz, 1 H, Manl-2), 5.09 (d, J = 8.1 Hz, 1 H, GaINAc-1), 4.96 (d, J = 3.5 Hz, 1 H, Glc-1), 4.91 (d, J = 10.9 Hz, 1 H), 4.88 (d, J = 1.6 Hz, 1 H), 4.83 (d, J = 10.6 Hz, 1 H), 4.74 - 4.56 (m, 13H), 4.54 - 4.29 (m, 8H), 4.28 - 4.21 (m, 2H), 4.20 - 4.15 (m, 2H), 4.10 - 3.82 (m, 16H), 3.79 - 3.48 (m, 13H), 3.33 (dt, J = 9.8, 6.3 Hz, 1 H), 3.24 (dd, J = 10.7, 1.6 Hz, 1 H), 3.18 (t, J = 6.9 Hz, 2H), 2.87 (dd, J = 10.5, 1.4 Hz, 1 H), 2.50 - 2.31 (m, 4H, CH₂ of Lev), 1.98 (s, 3H, Me), 1.57 - 1.45 (m, 4H), 1.38 - 1.26 (m, 2H), 1.17 - 1.01 (m, 21 H, TIPS); ¹³C NMR (151 MHz, CHCl₃) δ 206.13 (ketone of Lev), 172.17 (ester of Lev), 161.93 (C=O of amide), 139.12, 138.92, 138.88, 138.73, 138.64, 138.63, 138.58, 138.49, 138.36, 138.35, 138.28, 138.07, 138.00, 128.56, 128.52, 128.46, 128.43, 128.38, 128.34, 128.31, 128.29, 128.27, 128.26, 128.24, 128.22, 128.17, 128.14, 128.06, 128.00, 127.96, 127.91, 127.89, 127.85, 127.83, 127.79, 127.77, 127.75, 127.73, 127.71, 127.70, 127.66, 127.59, 127.55, 127.49, 127.44, 127.38, 127.33, 127.08, 100.14 (Glc-1, *J_{C,H}* = 170 Hz), 99.11 (GalNAc-1, *J_{C,H}* = 164 Hz), 99.00 (*J_{C,H}* = 174 Hz), 98.62 (*J_{C,H}* = 172 Hz), 97.30 (*J_{C,H}* = 172 Hz), 92.72 (CCl₃), 82.16, 81.10, 80.09, 79.77, 77.84, 76.79, 75.45, 75.38, 75.31, 75.18, 75.10, 75.03, 74.98, 74.62, 74.13, 74.07, 73.96, 73.62, 73.49, 73.31, 73.29, 72.56, 72.43, 72.36, 72.23, 71.78, 71.76, 71.63, 70.79, 70.17, 69.78, 69.63, 68.00, 67.60, 67.49, 66.49, 63.16, 56.23, 51.34, 37.89, 29.74, 28.90, 28.73, 28.13, 26.16, 23.53, 18.25, 18.23, 18.20, 18.16, 12.25, 12.21; ESI-MS: m/z [M+Na]⁺ cald for C₁₄₂H₁₆₅Cl₃N₄O₂₈Si 2533.0342, obsd 2533.0302.

### Example 7

### 1-O-5-Azidopentyl 2,3,4-tri-O-benzyl-α-D-mannopyranosyl-(1→2)-3,4,6-tri-O-benzyl-α-D-mannopyranosyl-(1→6)-3-O-benzyl-4-O-(2,3,4,6-tetra-O-benzyl-α-D-glucopyranosyl-(1→4)-3,6-di-O-benzyl-2-deoxy-2-trichloracetamido-β-D-galactopyranosyl)-2-O-levulinyl-α-D-mannopyranoside(8)

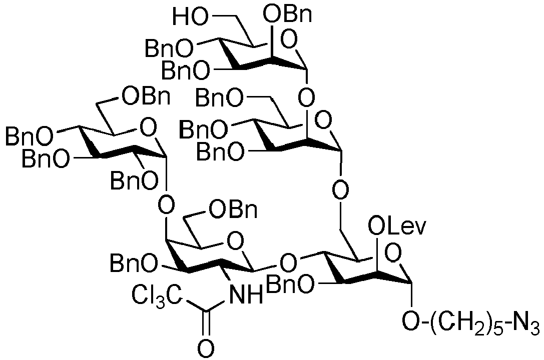

The pentasaccharide 7 of Example 6 (37 mg, 0.015 mmol) was dissolved in MeCN (1.5 mL) and water (2 drops). Sc(OTf)₃ (10.9 mg, 0.022 mmol) was added and the reaction mixture was heated to 50°C for 6 h. The reaction mixture was quenched with TEA (50 µL) and evaporated to dryness. The residue was purified by flash column chromatography to give alcohol 8 (33 mg, 0.014 mmol, 95% yield) as white foam: R_{f} (SiO₂, EtOAc/Hexane 1:2) = 0.24; [α]^{D}₂₀: + 43.0 (c = 1.00, CHCl₃); ATR-FTIR (cm⁻¹): 3030, 2920, 2097, 1717, 1454, 1362, 1072, 1028; ¹H NMR (400 MHz, CHCl₃) δ 7.61 (d, J = 7.1 Hz, 1 H), 7.35 - 7.11 (m, 60H), 7.09 (dd, J = 7.6, 1.8 Hz, 2H), 7.02 - 6.98 (m, 2H), 5.29 (d, J = 8.2 Hz, 1 H, GaINAc-1), 5.23 (dd, J = 3.4, 1.9 Hz, 1 H, ManI-2), 5.04 - 5.00 (m, 3H, ManII-1, ManIII-1, Glc-1), 4.91 - 4.78 (m, 3H), 4.76 - 4.60 (m, 10H), 4.59 - 4.48 (m, 6H), 4.43 (s, 2H), 4.40 - 4.30 (m, 3H), 4.13 - 3.96 (m, 9H), 3.92 (dd, J = 8.4, 3.5 Hz, 1 H, ManI-3), 3.89 - 3.56 (m, 18H), 3.55 - 3.49 (m, 2H), 3.38 - 3.25 (m, 3H), 3.18 (t, J = 6.9 Hz, 2H), 2.92 (dd, J = 10.8, 1.6 Hz, 1 H), 2.66 (t, J = 6.3 Hz, 1 H), 2.60 - 2.36 (m, 4H, CH₂ of Lev), 2.03 (s, 3H, Me of Lev), 1.60 - 1.47 (m, 4H), 1.41 - 1.31 (m, 2H); ¹³C NMR (101 MHz, CHCl₃) δ 206.43 (ketone of Lev), 172.13 (ester of Lev), 162.33 (C=O of amide), 138.94, 138.75, 138.74, 138.70, 138.68, 138.66, 138.53, 138.50, 138.47, 138.32, 138.13, 138.02, 128.59, 128.47, 128.45, 128.44, 128.43, 128.40, 128.37, 128.34, 128.18, 128.14, 128.02, 127.87, 127.85, 127.84, 127.83, 127.80, 127.70, 127.65, 127.57, 127.45, 127.33, 127.22, 126.89, 100.05, 99.94, 99.35, 99.32, 97.24 (ManI-1), 92.64, 82.01, 80.33, 80.05, 79.97, 77.85, 77.36, 76.29, 75.51, 75.47, 75.38, 75.27, 75.16, 75.05, 75.01, 74.88, 74.45, 74.15, 73.59, 73.50, 73.14, 73.11, 72.97, 72.70, 72.39, 72.36, 72.14, 71.88, 71.20, 70.77, 70.59, 69.36, 68.91 (ManI-2), 68.16, 67.77, 67.62, 67.49, 62.94, 56.91, 51.37, 37.97, 29.82, 28.91, 28.70, 28.17, 23.50; ESI-MS: m/z [M+Na]⁺ cald for C₁₃₃H₁₄₅Cl₃N₄O₂₈ 2375.9025, obsd 2375.9018.

### Example 8

### 1-O-5-Azidopentyl 2,3,4-tri-O-benzyl-α-D-mannopyranosyl-(1→2)-3,4,6-tri-O-benzyl-α-D-mannopyranosyl-(1→6)-3-O-benzyl-4-O-(2,3,4,6-tetra-O-benzyl-α-D-glucopyranosy)-(1→4)-3,6-di-O-benzyl-2-deoxy-2-trichloracetamido-β-D-galactopyranosyl)-α-D-mannopyranoside (9)

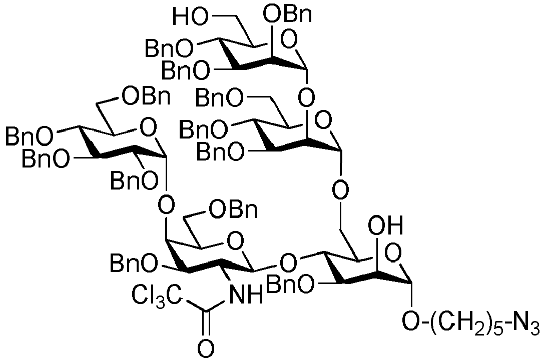

The pentasaccharide 8 of Example 7 (15 mg, 6.37 µmol) was dissolved in MeCN (1 mL) and hydrazine monohydrate (31 µL, 64 wt%) was added. The solution was stirred for 1 h before the reaction mixture was evaporated to dryness. The residue was purified by flash column chromatography to give diol 9 (8 mg, 3.55 µmol, 56% yield) as colorless oil: R_{f} (SiO₂, EtOAc/Hexane 3:1) = 0.68; [α]^{D}₂₀: + 42.3 (c = 0.80, CHCl₃); ATR-FTIR (cm⁻¹): 3411, 3030, 2924, 2096, 1713, 1454, 1041, 1027; ¹H NMR (400 MHz, CHCl₃) δ 7.34 - 7.07 (m, 63H), 6.94 - 6.89 (m, 2H), 5.13 (d, J = 8.2 Hz, 1 H, GalNAc-1), 5.08 (d, J = 1.7 Hz, 1 H), 5.03 (d, J = 1.6 Hz, 1 H), 5.01 (d, J = 3.3 Hz, 1 H, Glc-1), 4.88 (d, J = 11.0 Hz, 1 H), 4.82 (d, J = 10.7 Hz, 1 H), 4.78 - 4.73 (m, 3H), 4.69 (dd, J = 12.4, 6.4 Hz, 5H), 4.63 (d, J = 1.7 Hz, 1 H), 4.61 - 4.47 (m, 6H), 4.42 (t, J = 12.1 Hz, 4H), 4.32 (d,J = 11.6 Hz, 1H), 4.27 (d, J = 10.6 Hz, 1 H), 4.22 - 4.11 (m, 3H), 4.10 - 3.95 (m, 6H), 3.90 - 3.51 (m, 23H), 3.39 - 3.29 (m, 2H), 3.18 (t, J = 6.9 Hz, 2H), 2.93 (dd, J = 10.5, 1.6 Hz, 1 H), 2.70 (d, J = 1.5 Hz, 1 H), 2.44 (t, J = 6.6 Hz, 1 H), 1.58 - 1.47 (m, 4H), 1.40 - 1.30 (m, 2H); ¹³C NMR (101 MHz, CHCl₃) δ 162.05 (C=O of amide), 139.11, 138.84, 138.73, 138.68, 138.66, 138.49, 138.41, 138.31, 138.27, 138.20, 137.97, 128.72, 128.59, 128.55, 128.50, 128.47, 128.40, 128.37, 128.35, 128.29, 128.28, 128.21, 128.14, 128.03, 127.99, 127.86, 127.80, 127.78, 127.77, 127.74, 127.69, 127.66, 127.64, 127.62, 127.59, 127.56, 127.54, 127.51, 127.45, 127.14, 100.24, 99.73, 99.16, 98.78, 98.54, 92.65 (CCl₃), 82.04, 80.51, 80.34, 79.94, 78.12, 77.85, 76.37, 75.46, 75.33, 75.22, 75.13, 74.94, 74.65, 74.50, 73.61, 73.47, 73.27, 72.98, 72.52, 72.36, 72.27, 72.17, 71.81, 71.79, 70.96, 70.82, 69.39, 68.37, 68.01, 67.50, 67.28, 67.13, 62.89, 56.17, 51.40, 28.98, 28.76, 23.59; ESI-MS: m/z [M+Na]⁺ cald for C₁₂₈H₁₃₉Cl₃N₄O₂₆ 2277.8656, obsd 2277.8638.

### Example 9

### 1-O-5-Aminopentyl α-D-mannopyranosy)-(1→2)-α-D-mannopyranosy)-(1→6)-4-O-(α-D-glucopyranosyl-(1→4)-2-deoxy-2-acetamido-β-D-galactopyranosyl)-α-D-mannopyranoside (10)

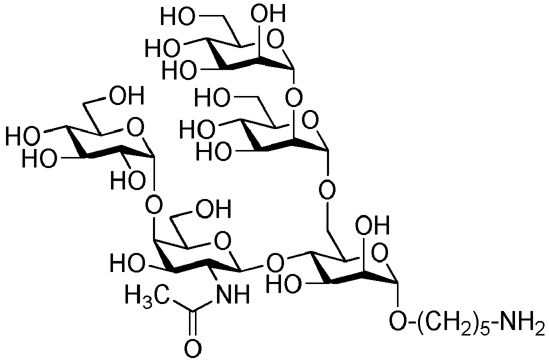

The diol 9 of Example 8 (8 mg, 3.55 µmol) was dissolved in MeOH (2 mL) and HOAc (2 drops). Pd/C (18.9 mg, 0.018mmol, 10 wt%) was added and hydrogen was bubbled through the slurry for 10 min. Afterwards the slurry was stirred for 24 h under hydrogen atmosphere before it was filtered through a syringe filter and evaporated to dryness. The residue was submitted to SEC chromatography (Sephadex-G25, 5% EtOH in water) to yield pentasaccharide 10 (2.5 mg, 2.62 µmol, 74%) as white solid: [α]^{D}₂₀: + 53.8 (c = 0.25, water); ATR-FTIR (cm⁻¹): 3271, 2930, 1643, 1558, 1407, 1018; ¹H NMR (600 MHz, D₂O) δ 5.24 (d, J = 1.1 Hz, 1 H), 5.09 (d, J = 1.1 Hz, 1 H), 5.01 (d, J = 3.8 Hz, 1 H, Glc-1), 4.92 (d, J = 1.3 Hz, 1 H), 4.61 (d, J = 8.3 Hz, 1 H, GaINAc-1), 4.18 - 4.15 (m, 1 H), 4.14 (dd, J = 2.9, 1.8 Hz, 1H), 4.11 (d, J = 2.7 Hz, 1 H), 4.09 - 3.70 (m, 26H), 3.66 - 3.61 (m, 2H), 3.54 (t, J = 9.7 Hz, 1 H), 3.07 (t, J = 7.6 Hz, 2H), 2.15 (s, 3H, Me), 1.79 - 1.69 (m, 4H), 1.57 - 1.46 (m, 2H); ¹³C NMR (151 MHz, D₂O) δ 184.10 (amide), 104.91, 104.73 (GaINAc-1), 102.86, 102.01 (Glc-1), 101.22, 81.26, 79.94, 79.31, 78.12, 75.89, 75.34, 74.60, 74.41, 72.99, 72.83, 72.72, 72.69, 72.61, 72.59, 72.15, 72.10, 71.85, 70.30, 69.60, 69.42, 68.70, 63.80, 63.71, 63.01, 62.57, 55.13, 30.67, 29.27, 25.20, 25.02; ESI-MS: m/z [M+H]⁺ cald for C₃₇H₆₆N₂O₂₆ 955.4, obsd 955.0.

### Example 10

### Triethylammonium 1-O-5-azidopentyl 2,3,4-tri-O-benzyl-6-O--(2-(N-benzyloxycarbonyl)aminoethyl-phosphonato)-α-D-mannopyranosyl-(1→2)-3,4,6-tri-O-benzyl-α-D-mannopyranosy)-(1→6)-3-O-benzy)-4-O-(2,3,4,6-tetra-O-benzyl-α-D-glucopyranosyl-(1→4)-3,6-di-O-benzyl-2-deoxy-2-trichloracetamido-β-D-galactopyranosyl)-α-D-mannopyranoside (11)

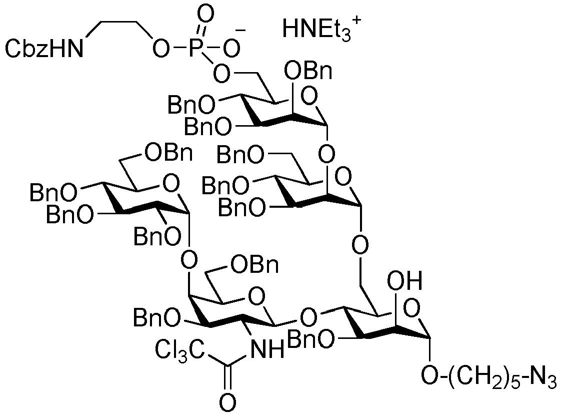

The pentasaccharide 8 of Example 7 (18 mg, 7.65 µmol) and triethylammonium 2-[(N-benzyloxycarbonyl)amino]ethyl H-phosphonate (9.7mg, 0.027mmol) were co evaporated with dry pyridine (3x2 mL) and placed under high vacuum for 30 min. The residue was dissolved in pyridine (2 mL) and PivCl (4.7 µL, 0.038mmol) was added. This solution was stirred for 2 h at r.t. before water (1 drop) and iodine (9.7 mg, 0.038mmol) were added. The solution was stirred for 1 h before hydrazine monohydrate (40 µL, 64 wt%) was added. The reaction mixture was stirred for 18 h before it was concentrated and purified by flash column chromatography to give phosphate 11 (16 mg, 6.12 µmol, 80% yield) as white foam: R_{f} (SiO₂, MeOH/CHCl₃ 1:9) = 0.63; [α]^{D}₂₀: + 46.1 (c = 1.6, CHCl₃); ATR-FTIR (cm⁻¹): 3030, 2925, 2095, 1712, 1497, 1454, 1052, 1028; ¹H NMR (400 MHz, CDCl₃) δ 12.01 (s, 1H, HNEt₃⁺), 7.42 (d, J = 7.2 Hz, 2H), 7.35 - 7.14 (m, 60H), 7.13 - 7.09 (m, 4H), 7.08 - 7.04 (m, 4H), 5.58 (s, 1 H, NH), 5.16 (s, 1 H), 5.06 - 4.95 (m, 3H), 4.91 - 4.81 (m, 3H), 4.81 - 4.74 (m, 2H), 4.72 - 4.59 (m, 6H), 4.59 - 4.41 (m, 9H), 4.40 - 4.28 (m, 3H), 4.23 (dd, J = 11.9, 7.0 Hz, 2H), 4.19 - 4.00 (m, 6H), 4.00 - 3.82 (m, 6H), 3.81 - 3.49 (m, 16H), 3.33 (d, J = 9.9 Hz, 1 H), 3.27 (dt, J = 10.1, 6.1 Hz, 1 H), 3.16 - 3.06 (m, 3H), 2.89 (dd, J = 10.8, 1.6 Hz, 1 H), 2.82 - 2.70 (m, 6H, CH₂ of TEA), 1.49 - 1.35 (m, 4H), 1.35 - 1.19 (m, 2H), 1.12 (t, J = 7.3 Hz, 9H, Me of TEA); ¹³C NMR (126 MHz, CDCl₃) δ 162.88 (amide), 156.44 (amide of Cbz), 139.27, 139.16, 139.11, 138.85, 138.75, 138.62, 138.55, 138.46, 138.30, 136.94, 128.63, 128.59, 128.45, 128.33, 128.25, 128.18, 128.12, 128.05, 127.83, 127.75, 127.69, 127.53, 127.28, 126.75, 101.45, 100.63, 98.45, 97.32, 93.16 (CCl₃), 82.23, 80.42, 80.26, 79.47, 78.90, 78.04, 75.61, 75.43, 75.24, 74.97, 74.58, 74.04, 73.82, 73.30, 73.17, 73.08, 72.74, 72.15, 71.78, 71.65, 71.06, 70.86, 70.32, 69.03, 67.74, 66.73, 66.61, 66.34, 65.39, 64.01, 54.86, 51.34, 45.59, 42.03, 29.83, 29.39, 28.74, 23.58, 22.83, 8.56; ESI-MS: m/z [M-H]⁻ cald for C₁₃₈H₁₅₁Cl₃N₅O₃₁P 2510.9190, obsd 2510.9392.

### Example 11

### 1-O-5-aminopentyl 6-O-(aminoethyl-phosphonato)-α-D-mannopyranosyl-(1 →2)-α-D-mannopyranosyl-(1→6)-4-O-(α-D-glucopyranosyl-(1→4)-2-deoxy-2-acetamido-β-D-galactopyranosyl)-α-D-mannopyranoside (12)

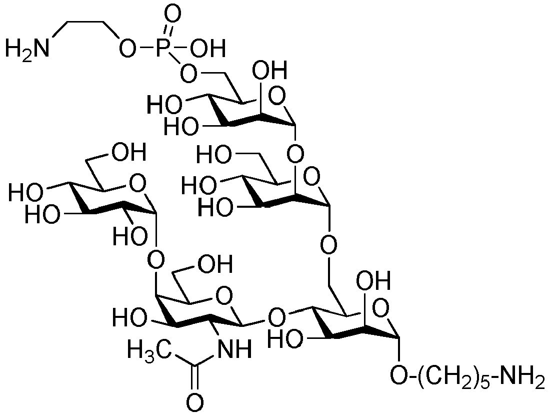

The phosphate 11 of Example 9 (16 mg, 6.12 µmol) was dissolved CHCl₃ (2 mL) and washed Amberlite IR120 H resin (50 mg) was added. The slurry was stirred for 45 min before it was filtered and concentrated. The residue was dissolved in MeOH (2 mL) and HOAc (2 drops). Pd/C (32.6 mg, 0.031 mmol, 10 wt%) was added and hydrogen was bubbled through the slurry for 10 min. Afterwards the slurry was stirred for 24 h under hydrogen atmosphere before it was filtered through a syringe filter and evaporated to dryness. The residue was submitted to SEC chromatography (Sephadex-G25, 5% EtOH in water) to yield phosphate 12 (4.8 mg, 4.45 µmol, 73%) as white solid: [α]^{D}₂₀: + 64.0 (c = 0.48, water); ATR-FTIR (cm⁻¹): 3335, 2484, 1634, 1020; ¹H NMR (400 MHz, D₂O) δ 5.22 (d, J = 1.4 Hz, 1 H), 5.06 (d, J = 1.5 Hz, 1 H), 4.99 (d, J = 3.9 Hz, 1H, Glc-1), 4.90 (d, J = 1.7 Hz, 1H), 4.58 (d, J = 8.3 Hz, 1H, GalNAc-1), 4.23 - 4.11 (m, 5H), 4.09 (d, J = 2.8 Hz, 1 H), 4.08 - 4.00 (m, 4H), 4.00 - 3.71 (m, 21 H), 3.66 - 3.58 (m, 2H), 3.52 (dd, J = 10.1, 9.3 Hz, 1 H), 3.34 (t, J = 4.9 Hz, 2H), 3.07 (t, J = 7.5 Hz, 2H), 2.14 (s, 3H, Me), 1.80 - 1.67 (m, 4H), 1.59 - 1.42 (m, 2H); ¹³C NMR (101 MHz, D₂O) δ 174.59 (C=O of amide), 102.46, 102.18 (GalNAc-1), 100.16 (Glc-1), 99.32, 98.15, 79.15, 77.49, 76.62, 75.39, 73.06, 72.66, 71.99, 71.89, 71.73, 70.21, 70.14, 69.94, 69.84, 69.47, 69.37, 69.16, 67.61, 66.88, 66.21, 66.04, 64.65, 61.83, 61.10, 60.33, 59.89, 52.41, 40.09, 40.02, 39.37, 28.01, 26.51, 22.56, 22.33; ³¹P NMR (162 MHz, D₂O) δ 0.36; ESI-MS: m/z [M+H]⁺ cald for C₃₉H₇₂N₃O₂₉P 1078.4, obsd 1078.4.

### B Preparation of Examples with R = H

### Example 12

### Allyl 3,4,6-tri-O-benzyl-2-deoxy-2-trichloracetamido-β-D-galactopyranosyl-(1→4)-3-O-benzyl2-O-levulinyl-6-O-tertbutyldiphenylsilyl-α-D-mannopyranoside (1a)

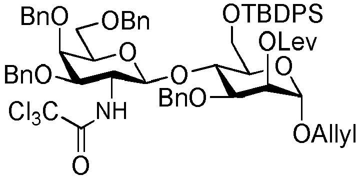

Dibutyl 3,4,6-tri-*O*-benzyl-2-deoxy-2-trichloracetamido-α-D-galactopyranosyl phosphate **A1** (63 mg, 0.080 mmol) and alcohol **B1** (40 mg, 0.062 mmol) were evaporated with toluene (3x2 mL) and placed under high vacuum for 30 min. The residue was dissolved in anhydrous DCM (3 mL) and molecular sieves (4A, 150 mg) were added. The slurry was stirred for 15 min at r.t. before it was cooled down to - 40°C. TMSOTf (14.5 µL, 0.080 mmol) was added dropwise and the yellow solution was stirred at -40°C for 1h before it was quenched with TEA (100 µL) and diluted with CHCl₃ (10 mL). The reaction mixture was filtered over Celite® and solvents were removed under reduced pressure. The residue was purified using flash column chromatography (*n-*hexane/ethyl acetate 5:1) to yield **1a** (62 mg, 0.051 mmol, 82% yield) as white foam: R_{f} (SiO₂, *n*-hexane/ethyl acetate 3:1) = 0.55; [α]²⁰_{D}: + 30.2 (c = 1.00, CHCl₃); ATR-FTIR (cm⁻¹): 3409, 3066, 3032, 2929, 2858, 1737, 1717, 1520, 1362, 1140, 1105, 1065cm⁻¹; ¹H NMR (600 MHz, CDCl₃) δ 7.77 - 7.71 (m, 4H), 7.43 - 7.24 (m, 21 H), 7.23 - 7.16 (m, 5H), 6.32 (d, *J* = 7.4 Hz, 1 H, NH), 5.93 - 5.84 (m, 1 H, CH of Allyl), 5.34 (dd, *J* = 3.3, 1.8 Hz, 1 H, Man-2), 5.26 (dd, *J* = 17.2, 1.5 Hz, 1 H, CH₂ of All), 5.20 (dd, *J* = 10.4, 1.2 Hz, 1 H, CH₂ of Allyl), 5.16 (d, *J* = 8.3 Hz, 1 H, Gal-1), 4.89 (d, *J* = 11.2 Hz, 1 H), 4.84 (d, *J* = 1.5 Hz, 1 H, Man-1), 4.69 - 4.62 (m, 3H), 4.57 (d, *J* = 11.2 Hz, 1 H), 4.47 (d, *J* = 11.3 Hz, 1 H), 4.35 (d, *J* = 11.8 Hz, 1 H), 4.27 (d, *J* = 11.8 Hz, 1 H), 4.18 (dd, *J* = 12.8, 5.3 Hz, 1 H), 4.09 - 4.03 (m, 2H), 4.01 - 3.94 (m, 4H), 3.86 (dd, *J* = 11.2, 5.4 Hz, 1 H), 3.77 (dd, *J* = 9.8, 4.9 Hz, 1 H), 3.70 (dt, *J* = 10.9, 7.9 Hz, 1 H), 3.63 (t, *J* = 8.6 Hz, 1 H), 3.45 (dd, *J* = 8.0, 5.4 Hz, 1 H), 3.33 (dd, *J* = 9.0, 5.2 Hz, 1 H), 2.65 - 2.53 (m, 2H, CH₂ of Lev), 2.53 - 2.46 (m, 1 H, CH₂ of Lev), 2.46 - 2.40 (m, 1 H, CH₂ of Lev), 2.05 (s, 3H, Me), 1.08 (s, 9H, tBu); ¹³C NMR (151 MHz, CDCl₃) δ 206.33 (ketone), 172.10 (ester), 161.89 (CCl₃CONH), 138.76, 138.70, 138.05, 137.80, 136.10, 135.73, 133.85, 133.62, 133.34, 129.98, 129.88, 128.57, 128.49, 128.32, 128.24, 128.13, 128.01, 127.97, 127.87, 127.84, 127.83, 127.72, 127.69, 127.20, 127.05, 117.96 (CH₂ of Allyl), 98.55 (Gal-1), 96.49 (Man-1), 92.39 (CCl₃CONH), 77.20, 76.52, 74.92, 73.56, 73.24, 72.91, 72.67, 72.21, 72.07, 71.66, 69.22, 68.16, 68.09, 63.70, 56.76, 38.06, 29.81 (Me of Lev), 28.20, 26.81 (Me of *t*Bu), 19.43 (C_{quart} of *t*Bu); (ESI-MS): *m*/*z* [M+Na]⁺ cald 1244.3893 for C₆₆H₇₄Cl₃NO₁₃Si, obsd 1244.3875.

### Example 13

### Allyl 3,4,6-tri-O-benzyl-2-deoxy-2-trichloracetamido-β-D-galactopyranosyl-(1→4)-3-O-benzyl-2-O-levulinyl-α-D-mannopyranoside (1b)

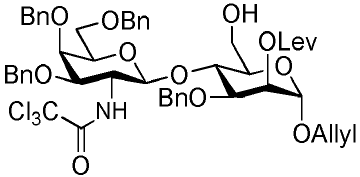

The disaccharide **1a** of Example 12 (62 mg, 0.051 mmol) was dissolved in THF (400 µL) in a 15 mL Falcon™ tube and HF-pyridine (101 µL, 5.570 mmol) was added. The solution was stirred for 24 h at room temperature before it was quenched with sat. NaHCO₃ solution and extracted with ethyl acetate (2 x 15 mL). Solvents were removed under reduced pressure and the residue was purified using flash column chromatography (*n*-hexane/ethyl acetate 1:2->1:3) to yield **1b** as yellow oil (40 mg, 0.041 mmol, 80% yield): R_{f} (SiO₂, *n*-hexane/ethyl acetate 1:3) = 0.24; [α]²⁰_{D}: + 26.2 (c = 1.00, CHCl₃); ATR-FTIR (cm⁻¹): 3506, 3332, 3062, 2923, 2870, 1740, 1718, 1530, 1455, 1364, 1139, 1100, 1070, 1027; ¹H NMR (600 MHz, CDCl₃) δ 7.27 - 7.08 (m, 20H), 6.84 (d, *J* = 7.3 Hz, 1 H, NH), 5.82 - 5.73 (m, 1 H, CH of Allyl), 5.24 (dd, *J* = 3.2, 1.9 Hz, 1 H, Man-2), 5.18 (dd, *J* = 17.2, 1.5 Hz, 1 H, CH₂= of Allyl), 5.14 - 5.10 (m, 2H, CH₂= of Allyl and Gal-1), 4.81 (d, *J* = 11.4 Hz, 1 H), 4.74 (d, *J* = 1.4 Hz, 1 H, Man-1), 4.61 (d, *J* = 11.6 Hz, 1 H), 4.55 (d, *J* = 11.2 Hz, 1 H), 4.48 (d, *J* = 11.5 Hz, 2H), 4.40 (d, *J* = 11.2 Hz, 1 H), 4.31 (d, *J* = 11.7 Hz, 1 H), 4.22 (d, *J* = 11.7 Hz, 1 H), 4.11 (dd, *J* = 10.9, 2.4 Hz, 1 H), 4.08 - 4.02 (m, 2H), 3.93 - 3.84 (m, 4H), 3.75 (dd, *J* = 12.1, 3.4 Hz, 1 H), 3.72 - 3.68 (m, 1 H), 3.62 - 3.57 (m, 1 H), 3.55 - 3.49 (m, 2H), 3.41 - 3.35 (m, 1 H), 2.52 - 2.38 (m, 3H, CH₂ of Lev), 2.33 (dt, *J* = 16.7, 6.2 Hz, 1 H, CH₂ of Lev), 1.99 (s, 3H, Me of Lev).; ¹³C NMR (151 MHz, CDCl₃) δ 206.47 (ketone), 172.00 (ester), 162.17 (amide), 138.63, 138.27, 137.88, 137.66, 133.42, 128.58, 128.54, 128.46, 128.35, 128.10, 128.06, 128.03, 127.94, 127.70, 127.45, 126.86, 118.05 (CH₂ = of Allyl), 98.63 (Gal-1), 96.80 (Man-1), 92.73 (CCl₃CONH), 77.24, 76.45, 74.87, 73.61, 73.50, 72.24, 71.97, 71.71, 71.07, 68.87, 68.43, 68.40, 61.76, 56.29, 38.00, 29.82, 29.80 (Me of Lev), 28.11.; ESI-MS for C₅₀H₅₆Cl₃NO₁₃: m/z [M+Na]⁺ cald 1006.2715, obsd 1006.2714.

### Example 14

### Allyl 2,3,4-tri-O-benzyl-6-O-triisopropylsilyl-α-D-mannopyranosyl-(1→2)-3,4,6-tri-O-benzyl-α-D-mannopyranosyl-(1→6)-3-O-benzyl-4-O-(3,4,6-tri-O-benzyl-2-deoxy-2-trichloracetamido-β-D-galactopyranosyl)-2-O-levulinyl-α-D-mannopyranoside (14a)

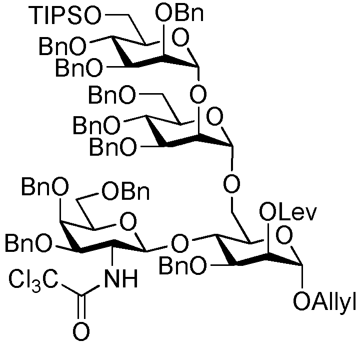

The disaccharide **1a** of Example 13 (40 mg, 0.041 mmol) and dimannoside **13** (63 mg, 0.054 mmol) were co-evaporated with toluene (3 x 2 mL) and placed under high vacuum for 30 min. The residue was dissolved in anhydrous diethyl ether (3 mL) and molecular sieves (4A, 150 mg) were added. The slurry was stirred for 10 min at room temperature before it was cooled down to 0°C and TBSOTf (9.3 µL, 0.041 mmol) was added. The reaction mixture was stirred for 1 h at 0°C before it was quenched with TEA (100 µL) and filtered over Celite®. Solvents were evaporated under reduced pressure and the crude product was purified using flash column chromatography (*n-*hexane/ethyl acetate 4:1) to yield tetrasaccharide **14a** (60 mg, 0.030 mmol, 74% yield) as yellow oil: R_{f} (SiO₂, *n-*hexane/ethyl acetate 4:1) = 0.24; [α]²⁰_{D}: + 19.8 (c = 1.00, CHCl₃); ATR-FTIR (cm⁻¹): 3350, 2925, 2866, 1719, 1524, 1454, 1363, 1137, 1100, 1071, 1028; ¹H NMR (400 MHz, CDCl₃) δ 7.26 - 7.04 (m, 50H), 6.43 (s, 1 H, NH {rotamer}), 6.08 (s, 1 H, NH {rotamer}), 5.73 (ddd, *J* = 22.2, 10.9, 5.8 Hz, 1 H, CH of Allyl), 5.26 - 5.21 (m, 2H, Manl-2{5.24ppm}), 5.15 (d, *J* = 17.2 Hz, 1 H, CH₂= of Allyl), 5.08 (d, *J* = 8.3 Hz, 1 H, Gal-1), 5.05 (d, *J* = 10.5 Hz, 1 H, CH₂= of Allyl), 4.83 (d, *J* = 10.9 Hz, 1 H), 4.77 - 4.69 (m, 3H), 4.68 (d, *J* = 1.3 Hz, 1 H, Manl-1), 4.64 - 4.22 (m, 16H), 4.15 (d, *J* = 11.7 Hz, 1 H), 4.10 - 3.93 (m, 5H), 3.91 - 3.53 (m, 17H), 3.53 - 3.40 (m, 2H), 3.29 (dd, *J* = 8.2, 4.7 Hz, 1 H), 2.51 - 2.29 (m, 4H, CH₂ of Lev), 1.96 (s, 3H, Me of Lev), 1.04 - 0.98 (m, 21 H, TIPS); ¹³C NMR (101 MHz, CDCl₃) δ 206.33 (ketone), 172.02 (ester), 163.47 (amide {rotamer}), 162.19 (amide {rotamer}), 139.10, 138.89, 138.65, 138.58, 138.55, 138.34, 138.17, 137.99, 137.71, 133.33, 128.54, 128.48, 128.46, 128.44, 128.38, 128.34, 128.28, 128.19, 128.14, 128.08, 128.07, 128.05, 127.93, 127.88, 127.84, 127.79, 127.75, 127.73, 127.62, 127.57, 127.54, 127.45, 127.40, 127.36, 127.26, 118.43 (CH₂= of Allyl), 98.68 (Gal-1), 98.55, 98.36, 96.33 (Manl-1), 92.71 (CCl₃CO), 80.90, 79.67, 77.36, 76.38, 75.26, 75.18, 74.98, 74.95, 74.72, 74.53, 73.85, 73.60, 73.47, 73.37, 72.43, 72.27, 72.19, 72.13, 71.70, 71.39, 69.99, 68.87 (Manl-2), 68.43, 67.99, 66.41, 63.03, 56.45, 37.99 (CH₂ of Lev), 29.82 (Me of Lev), 28.13 (CH₂ of Lev), 18.22 (TIPS), 18.17 (TIPS), 12.19 (TIPS); (ESI-MS): m/z [M+Na]⁺ cald 2026.7917 for C₁₁₃H₁₃₂Cl₃NO₂₃Si, obsd 2026.7891.

### Example 15

### Allyl 2,3,4-tri-O-benzyl-6-O-triisopropylsilyl-α-D-mannopyranosyl-(1→2)-3,4,6-tri-O-benzy)-α-D-mannopyranosy)-(1→6)-4-O-(3,4,6-tri-O-benzyl-2-deoxy-2-acetamido-β-D-galactopyranosyl)-3-O-benzyl-2-O-levulinyl-α-D-mannopyranoside (14)

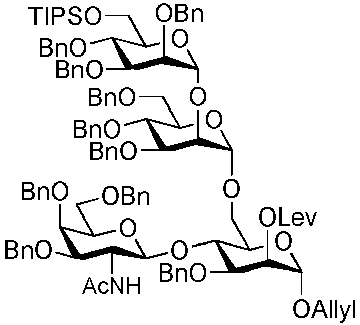

The tetrasaccharide **14a** of Example 14 (60 mg, 0.030 mmol) was dissolved in acetic acid (1 mL) and heated to 55°C to give a colorless solution. Zinc (195 mg, 2.99 mmol) was added to this solution in three portions over 30 min. Afterwards the slurry was stirred for 2 h at 55°C before it was filtered, diluted with toluene (10 mL) and evaporated to dryness. The residue was purified using column chromatography (*n-*hexane/ethyl acetate 2:1) to yield tetrasaccharide **14** (42 mg, 0.022 mmol, 74% yield) as yellow oil: R_{f} (SiO₂ *n-*hexane/ethyl acetate 2:1) = 0.22; [α]²⁰_{D}: + 22.6 (c = 1.00, CHCl₃); ATR-FTIR (cm⁻¹): 3349, 2924, 2865, 1741, 1721, 1675, 1454, 1364, 1136, 1064, 1028cm⁻¹; ¹H NMR (400 MHz, CDCl₃) δ 7.30 - 7.03 (m, 50H), 6.21 (d, J = 7.4 Hz, 1 H, NH), 5.74 (dq, J = 10.7, 5.7 Hz, 1 H, CH of Allyl), 5.28 - 5.11 (m, 3H), 5.06 (t, J = 8.6 Hz, 2H), 4.85 (d, J = 10.8 Hz, 1 H), 4.79 - 4.67 (m, 4H), 4.64 - 4.28 (m, 14H), 4.23 - 4.15 (m, 2H), 4.13 - 3.36 (m, 25H), 3.16 (dd, J = 8.2, 4.7 Hz, 1 H), 2.51 - 2.31 (m, 4H, CH₂ of Lev), 1.96 (s, 3H, Me of Lev), 1.69 (s, 3H, Me of NHAc), 1.08 - 0.93 (m, 21 H, TIPS); ¹³C NMR (101 MHz, CDCl₃) δ 206.26 (ketone), 172.12 (ester), 170.95 (amide), 139.08, 138.93, 138.70, 138.57, 138.39, 138.30, 138.08, 137.80, 133.28 (CH of Allyl), 128.59, 128.54, 128.43, 128.41, 128.36, 128.32, 128.30, 128.21, 128.15, 128.14, 128.09, 128.00, 127.94, 127.89, 127.78, 127.74, 127.61, 127.59, 127.47, 127.37, 127.29, 127.18, 118.38 (**C**H₂= of Allyl), 100.33, 98.43, 98.11, 96.50 (4x anomeric carbon), 80.78, 79.55, 78.31, 76.49, 75.51, 75.23, 75.04, 74.57, 74.50, 74.09, 73.98, 73.50, 73.36, 73.31, 72.40, 72.35, 72.28, 72.14, 71.81, 71.76, 71.47, 70.19, 69.48, 68.95, 68.59, 68.03, 65.83, 64.68, 63.03, 55.03, 37.98 (**C**H₂ of Lev), 29.82 (**C**H₃ of Lev), 28.14 (**C**H₂ of Lev), 23.55 (**C**H₃CONH), 18.22 (TIPS), 18.17 (TIPS), 12.20 (TIPS); ESI-MS for C₁₁₃H₁₃₅NO₂₃Si: m/z [M+Na]⁺ cald 1925.9125, obsd 1925.9080.

### Example 16

### 2,3,4-tri-O-benzyl-6-O-triisopropylsilyl-α-D-mannopyranosyl-(1 →2)-3,4,6-tri-O-benzyl-α-D-mannopyranosyl-(1 →6)- 4-O-(3,4,6-tri-O-benzyl-2-deoxy-2-acetamido-β-D-galactopyranosyl)-3-O-benzyl-2-O-levulinyl-α-D-mannopyranose (15)

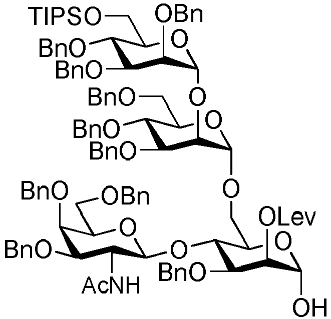

Hydrogen was bubbled for 20 min through a solution of [IrCOD(PPh₂Me)₂]PF₆ (10 mg, 0.012 mmol) in anhydrous THF (3 mL) to yield a pale yellow solution. The atmosphere was exchanged to nitrogen and the solution was added to the tetrasaccharide **14** of Example 15 (40 mg, 0.021 mmol). The resulting reaction mixture was stirred for 12 h at room temperature before removal of solvents. The residue was dissolved in acetone (2.8 mL) and water (0.2 mL). HgCl₂ (34.2 mg, 0.126 mmol) and HgO (0.5 mg, 0.002 mmol) were added and the solution was stirred for 2 h at room temperature before it was diluted with ethyl acetate (15 mL) and washed with sat. NaHCO₃ solution (15 mL). The organic layer was dried over Na₂SO₄, filtered and concentrated. The residue was purified using column chromatography (*n-*hexane/ethyl acetate 2:1) to yield lactol **15** (27 mg, 0.014 mmol, 69% yield) as colorless oil: R_{f} (SiO₂, *n-*hexane/ethyl acetate 3:2) = 0.20; [α]²⁰_{D}: + 15.3 (c = 1.00, CHCl₃); ATR-FTIR (cm⁻¹): 3349, 2925, 2865, 1739, 1720, 1673, 1455, 1364, 1105, 1067, 1028; ¹H NMR (600 MHz, CDCl₃) δ 7.29 (d, *J* = 7.3 Hz, 2H), 7.26 - 7.10 (m, 44H), 7.07 - 7.02 (m, 4H), 6.28 (d, *J* = 7.1 Hz, 1 H, NH), 5.12 (d, *J* = 8.3 Hz, 1 H, GalNAc-1), 5.04 (s, 1 H), 4.92 (s, 1 H), 4.83 (d, *J* = 10.3 Hz, 1 H), 4.76 (d, J = 3.8 Hz, 1H), 4.71 (d, *J* = 11.6 Hz, 1 H), 4.63 - 4.40 (m, 12H), 4.35 (dd, *J* = 24.0, 11.8 Hz, 3H), 4.26 (dd, *J* = 11.4, 1.6 Hz, 2H), 4.14 - 3.93 (m, 8H), 3.87 - 3.74 (m, 5H), 3.67 (d, *J* = 6.5 Hz, 2H), 3.58 - 3.50 (m, 3H), 3.48 - 3.41 (m, 4H), 3.37 (dd, J = 8.1, 5.3 Hz, 1 H), 3.30 (dd, J = 8.4, 6.6 Hz, 1 H), 3.04 (dd, *J* = 8.7, 5.0 Hz, 1 H), 2.53 - 2.35 (m, 4H, CH₂ of Lev), 1.99 (s, 3H, CH₃ of Lev), 1.73 (s, 3H, C**H**₃CONH), 1.05 - 0.99 (m, 21 H, TIPS); ¹³C NMR (151 MHz, CDCl₃) δ 206.39 (ketone), 171.90 (ester), 171.24 (amide), 139.01, 138.94, 138.89, 138.67, 138.42, 138.20, 138.17, 138.13, 137.60, 128.71, 128.59, 128.57, 128.45, 128.43, 128.30, 128.24, 128.21, 128.11, 128.10, 128.03, 127.98, 127.97, 127.95, 127.90, 127.74, 127.70, 127.69, 127.63, 127.45, 127.45, 127.16, 126.70, 100.46 (GalNAc-1; *J_{CH}* = 167Hz; (3), 97.88 (*J_{CH}* = 173Hz; α), 96.81 (*J_{CH}* = 172Hz; α), 91.86 (*J_{CH}* = 176Hz, α), 79.82, 77.78, 77.37, 77.16, 76.95, 76.65, 75.99, 75.79, 75.41, 75.15, 74.89, 74.64, 73.39, 73.36, 73.12, 72.71, 72.59, 72.51, 72.33, 72.26, 72.07, 72.01, 70.96, 70.07, 69.75, 69.01, 68.32, 67.58, 62.90, 55.93, 38.11 (CH₂ of Lev), 29.89 (CH₃ of Lev), 28.22 (CH₂ of Lev), 23.54 (**C**H₃CONH), 18.25 (TIPS), 18.19 (TIPS), 12.26 (TIPS); (ESI-MS): m/z [M+Na]⁺ cald 1885.8806 for C₁₁₀H₁₃₁NO₂₃Si, obsd 1885.8787.

### Example 17

### 2,3,4-Tri-O-benzyl-6-O-triisopropylsilyl-α-D-mannopyranosyl-( →2)-3,4,6-tri-O-benzyl-α-D-mannopyranosyl-(1 →6)-4-O-(3,4,6-tri-O-benzyl-2-deoxy-2-acetamido-β-D-galactopyranosyl)-3-O-benzyl-2-O-levulinyl-α-D-mannopyranosyl-(1→4)-2-azido-3,6-di-O-benzyl-2-deoxy-α-D-glucopyranosyl-(1 →6)-1-O-alkyl-2,3,4,5-tetra-O-benzyl-D-myo-inositol (16)

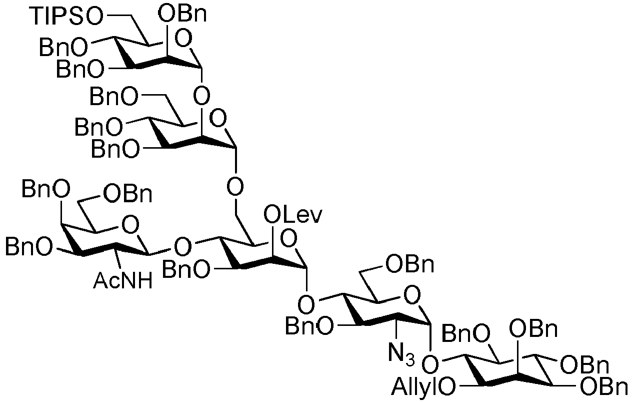

The lactol **15** of Example 16 (27 mg, 0.014 mmol) was dissolved in anhydrous DCM (1mL) and trichloroacetonitrile (145 µL, 1.449 mmol) was added. Afterwards DBU (1.1 µL, 0.007 mmol) was added and the reaction mixture was stirred for 1 h at room temperature before removal of solvents. The residue was purified using column chromatography (*n-*hexane/ethyl acetate 2:1 containing 0.1% TEA) to yield imidate **16a** (26 mg, 0.013 mmol, 89% yield) as an inseparable mixture of anomers. Imidate **16a** (26 mg, 0.013 mmol) and pseudodisaccharide **18** (10 mg, 0.011 mmol) were evaporated with toluene (3 x 2 mL) and the residue was placed under high vacuum for 30min. The residue was dissolved in anhydrous diethyl ether (1 mL) and molecular sieves (4A, 50 mg) were added. The slurry was stirred for 10 min at room temperature before it was cooled down to 0°C and TBSOTf (2.4 µL, 0.011 mmol) was added. The reaction mixture was stirred for 1 h at 0°C before it was quenched with TEA (50 µL) and filtered over Celite®. Solvents were removed under reduced pressure and the crude product was purified using flash column chromatography (*n-*hexane/ethyl acetate 3:1) to yield pseudohexasaccharide **16** (24 mg, 0.009 mmol, 81 % yield) as yellow oil: R_{f} (SiO₂, *n-*hexane/ethyl acetate 2:1) = 0.51; [α]²⁰_{D}: + 25.3 (c = 1.00, CHCl₃); ATR-FTIR (cm⁻¹) 3353, 3088, 3064, 3031, 2924, 2864, 2106, 1741, 1721, 1676, 1454, 1362, 1095, 1055, 1028, 913; ¹H NMR (400 MHz, CDCl₃) δ 7.34-7.02 (m, 80H), 5.94 (d, *J* = 8.8 Hz, 1 H, NH), 5.84 (m, 1 H, vinylic), 5.62 (d, *J* = 3.6 Hz, 1 H, Glc-1), 5.29-5.28 (m, 2H, Manl-1, Manl-2), 5.18 (dd, *J* = 1.5, 17.2 Hz, 1 H, =CH₂), 5.09-5.07 (m, 2H, anomeric and =CH₂), 4.92 (d, *J* = 11.2 Hz, 1 H, -CH₂Ph), 4.84 (d, *J* = 10.6 Hz, 1 H, -CH₂Ph), 4.83 (d, *J* = 10.6 Hz, 1 H, -CH₂Ph), 4.79-3.13 (m, 68H), 2.21-2.12 (m, 4H, -CH₂- of Lev), 1.82 (s, Me of Lev, 3H), 1.57 (s, Me of -NHAc, 3H), 0.97 (br, 21 H, TIPS); ¹³C NMR (101 MHz, CDCl₃) δ 205.84 (ketone of Lev), 171.75 (ester of Lev), 170.30 (amide of -NHAc), 138.97, 138.90, 138.86, 138.78, 138.61, 138.51, 138.48, 138.43, 138.30, 138.26, 138.20, 138.13, 137.97, 137.85, 137.63, 134.34 (vinylic of allyl), 128.57, 128.54, 128.52, 128.50, 128.46, 128.40, 128.37, 128.34, 128.28, 128.25, 128.23, 128.21, 128.15, 128.06, 128.03, 127.98, 127.91, 127.79, 127.74, 127.71, 127.67, 127.63, 127.59, 127.58, 127.54, 127.52, 127.43, 127.39, 127.31, 127.23, 127.13, 126.88, 117.18 (=CH₂ of allyl), 100.80 (GalNHAc-1), 99.39 (Manll-1), 98.55 (Manlll-1), 98.38 (Manl-1), 97.51 (Glc-11), 82.03, 81.86, 81.23, 80.94, 80.58, 79.79, 79.17, 75.79, 75.65, 75.52, 75.34, 75.28, 75.05, 74.51, 74.32, 74.26, 74.09, 73.88, 73.80, 73.57, 73.50, 73.21, 72.90, 72.82, 72.64, 72.36, 72.25, 72.18, 72.04, 71.51, 71.42, 71.11, 70.90, 70.71, 69.90, 69.76, 68.98, 68.39, 66.64, 63.74, 62.87, 53.09, 37.82 (-CH₂- of Lev), 29.64 (-CH₃), 28.00 (-CH₂- of Lev), 23.19 (-CH₃), 18.17 (-CH₃ of TIPS), 18.12 (-CH₃ of TIPS), 12.11 (-CHMe₂ of TIPS); ESI-HRMS: m/z [M+Na]⁺ cald 2814.3025, obsd 2814.3069.

| 400/700 MHz NMR Data (n.d. = not determined) | | **16** | | | | |
|---|---|---|---|---|---|---|
| | | GlcN | Manl | Manll | Manlll | GalNAc |
| chemical shift (ppm) | ¹H | 5.62 | 5.29 | 4.54 | 5.09 | 4.71 |
| | ¹³C | 97.51 | 98.38 | 99.39 | 98.55 | 100.80 |
| coupling constant (Hz) | H-H | 3.6 | n.d. | n.d. | n.d. | n.d. |
| | C-H | 178 | 174 | 172 | 171 | 159 |

### Example 18

### 2,3,4-Tri-O-benzyl-6-O-triisopropylsilyl-α-D-mannopyranosyl-(1 →2)-3,4,6-tri-O-benzyl-α-D-mannopyranosyl-(1 →6)-4-O-(3,4,6-tri-O-benzyl-2-deoxy-2-acetamido-β-D-galactopyranosyl)-3-O-benzyl-2-O-levulinyl-α-D-mannopyranosyl-(1 →4)-2-azido-3,6-di-O-benzyl-2-deoxy-α-D-glucopyranosyl-(1→6)-1-O-allyl-2,3,4,5-tetra-O-benzyl-D-myo-inosito (17)

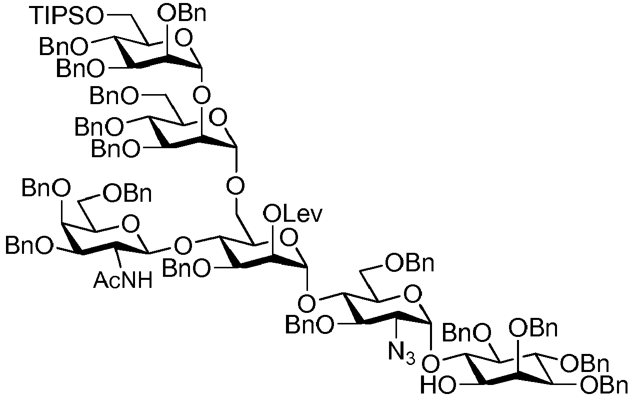

A solution of [IrCOD(PPh₂Me)₂]PF₆ (25 mg, 0.030 mmol) in THF (2 mL) was stirred under hydrogen atmosphere until the color turned from red to colorless to pale yellow (ca. 10 min). The hydrogen atmosphere was exchanged with argon, the pseudohexasaccharide (0.870 g, 0.311 mmol) **16** of Example 17 in THF (5 mL) was added. After 40 h, the solvent was removed and the residue was dissolved in acetone (6.5 mL). Water (0.5 mL), mercury(II) chloride (0.423 g, 1.56 mmol) and mercury(II) oxide (7.0 mg, 0.032 mmol) were added. After 20 min, NaHCO_{3(aq)} (15 mL) was added and the reaction mixture was extracted with DCM (3 x 10 mL). The combined organic layers were dried over Na₂SO₄, filtered and concentrated. The crude product was purified by silica gel column chromatography to give alcohol **17** (0.70 g, 0.254 mmol, 82% yield) as colorless oil: R_{f} (SiO₂, *n-*hexane/ethyl acetate 2:1) = 0.21 [α]²⁰_{D}: + 24.6 (c = 0.71, CHCl₃); ATR-FTIR (cm⁻¹) 3349, 3089, 3064, 3031, 2924, 2865, 2107, 1739, 1720, 1675, 1454, 1362, 1094, 1053, 1028; ¹H NMR (400 MHz, CDCl₃) δ 7.30-7.02 (m, 80H), 6.39 (br, 1 H, OH), 5.94 (d, *J* = 8.6 Hz, 1 H, NH), 5.28 (d, *J* = 3.5 Hz, 1 H, Glc-1), 5.27-5.26 (m, 2H, Manl-1, Manl-2), 5.10 (br, 1 H, Manlll-1), 4.90 (d, *J* = 11.6 Hz, 1 H, -CH₂-), 4.88 (d, *J* = 10.8 Hz, 1 H, -CH₂-), 4.83 (d, *J* = 10.7 Hz, 1 H, -CH₂-), 4.83 (d, *J* = 10.7 Hz, 1 H, -CH₂-), 4.75-4.35 (m, 20H, GaINAc-1, Manll-1, -CH₂-*18), 4.27-4.19 (m, 7H, -CH₂-*7), 4.10-3.76 (m, 17H), 3.74-3.68 (m, 2H), 3.63-3.26 (m, 17H), 2.19-3.11 (m, 2H), 2.23-2.15 (m, 4H, -CH₂- of Lev), 1.82 (s, Me of Lev, 3H), 1.57 (s, Me of -NHAc, 3H), 0.99-0.96 (m, 21 H, TIPS); ¹³C NMR (101 MHz, CDCl₃) δ 205.91 (ketone of Lev), 171.74 (ester of Lev), 170.52 (amide of - NHAc), 138.96, 138.91, 138.88, 138.72, 138.64, 138.49, 138.47, 138.42, 138.30, 138.26, 138.20, 137.98, 137.71, 137.61, 128.56, 128.55, 128.53, 128.50, 128.49, 128.44, 128.39, 128.37, 128.32, 128.27, 128.25, 128.19, 128.17, 128.08, 128.03, 127.98, 127.95, 127.84, 127.79, 127.77, 127.74, 127.70, 127.67, 127.64, 127.57, 127.51, 127.43, 127.31, 127.03, 100.76 (GalNHAc-1), 99.43 (Manll-1), 98.58 (Manlll-1), 98.22 (Manl-1), 98.04 (Glc-1), 82.00, 81.38, 81.00, 80.97, 80.92, 80.57, 79.53, 79.26, 77.23, 75.86, 75.79, 75.73, 75.31, 75.04, 74.91, 74.57, 74.38, 74.21, 73.89, 73.84, 73.58, 73.51, 73.30, 73.00, 72.79, 72.59, 72.39, 72.28, 72.22, 72.08, 71.58, 71.54, 70.73, 70.58, 69.85, 69.00, 68.57, 66.54, 64.61, 62.97, 53.33, 37.81 (-CH₂- of Lev), 29.69 (-CH₃), 28.01 (-CH₂- of Lev), 23.25 (-CH₃), 18.22 (-CH₃ of TIPS), 18.17 (-CH₃ of TIPS), 12.15 (-CHMe₂ of TIPS); ESI-MS: m/z [M+Na]⁺ cald 2775.2751, obsd 2775.2783.

### Example 19

### Triethylammonium 2,3,4-tri-O-benzyl-6-O-triisopropylsilyl-α-D-mannopyranosyl-(1→2)-3,4,6-tri-O-benzyl-α-D-mannopyranosyl-(1→6)-4-O-(3,4,6-tri-O-benzyl-2-deoxy-2-acetamido-β-D-galactopyranosyl)-3-O-benzyl-2-O-levulinyl-α-D-mannopyranosyl-(1→4)-2-azido-3,6-di-O-benzyl-2-deoxy-α-D-glucopyranosyl-(1→6)-2,3,4,5-tetra-O-benzyl-1-O-(1,2-di-O-octadecanoyl-sn-glyceryl-phosphonato)-D-myo-inositol (19a)

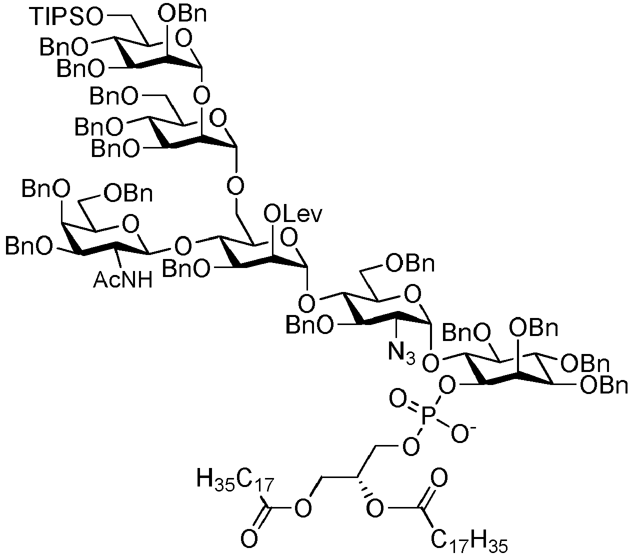

The alcohol **17** of example 18 (48 mg, 0.017 mmol) and triethylammonium (*S*)-2,3-bis(stearoyloxy)propyl phosphonate (20.7 mg, 0.026 mmol) were co-evaporated with anhydrous pyridine (3 x 2 mL) and placed under high vacuum for 30 min. The residue was dissolved in anhydrous pyridine (2 mL) and PivCl (5.4 µL, 0.044 mmol) was added. The reaction mixture was stirred for 2 h at room temperature before water (50 µL) and iodine (8.9 mg, 0.035 mmol) were added. The reaction mixture was stirred for 1 h before it was quenched with sat. Na₂S₂O_{3(aq)} dried over Na₂SO₄ and filtered. Solvents were evaporated and the residue was co-evaporated with toluene (3 x 2 mL). The crude product was purified using flash column chromatography (CHCl₃/MeOH from 100:0 -> 96:4). The fractions containing the desired product were pooled and washed with TEA/CO₂-Buffer (2x15 mL), dried over Na₂SO₄ and concentrated to yield lipidated pseudohexasaccharide **19a** (56 mg, 0.016 mmol, 91 % yield) as yellow oil: R_{f} (SiO₂, CHCl₃/MeOH 9:1) = 0.53; [α]²⁰_{D}: + 30.8 (c = 1.00, CHCl₃); ATR-FTIR (cm⁻¹): 3089, 3031, 2924, 2854, 2108, 1739, 1678, 1454, 1362, 1094, 1052, 1028; ¹H NMR (600 MHz, CDCl₃) δ 7.44 (d, *J* = 7.7 Hz, 2H), 7.38 - 7.12 (m, 76H), 7.08 (dd, *J* = 6.6, 2.8 Hz, 2H), 6.03 (d, *J* = 8.8 Hz, 1 H, NH), 5.90 (d, *J* = 3.7 Hz, 1 H, GlcNH₂-1), 5.36 - 5.33 (m, 2H, Manl-2), 5.25 (td, *J* = 8.8, 5.7 Hz, 1 H, CH of glycerol), 5.17 (d, *J* = 1.0 Hz, 1 H), 5.04 (d, *J* = 11.9 Hz, 1 H), 4.98 - 4.91 (m, 3H), 4.90 - 4.74 (m, 9H), 4.73 - 4.58 (m, 6H), 4.57 - 4.48 (m, 4H), 4.46 - 4.28 (m, 12H), 4.19 - 4.00 (m, 12H), 4.00 - 3.96 (m, 1 H), 3.94 - 3.85 (m, 4H), 3.83 (dd, *J* = 10.8, 3.1 Hz, 1 H), 3.78 (dd, *J* = 9.4, 7.1 Hz, 1 H), 3.71 - 3.39 (m, 16H), 3.36 - 3.32 (m, 2H), 3.13 (dd, *J* = 10.2, 3.7 Hz, 1 H, GlcNH₂-2), 2.90 (dd, *J* = 14.0, 6.8 Hz, 6H, CH₂ of TEA), 2.32 - 2.19 (m, 8H, CH₂ of Lev; 2x O-COC**H**₂-CH₂), 1.92 (s, 3H, Me of Lev), 1.63 (s, 3H, Me of NHAc), 1.59 - 1.52 (m, 4H, 2x O-COCH₂-C**H**₂), 1.33 - 1.23 (m, 56H, CH₂ of lipid), 1.21 (t, *J* = 7.3 Hz, 9H, Me of TEA), 1.10 - 1.05 (m, 21 H, TIPS), 0.89 (t, *J* = 7.0 Hz, 6H, Me of lipid); ¹³C NMR (126 MHz, CDCl₃) δ 205.90 (ketone), 173.46, 173.10 (2 x fatty acid ester), 171.77 (ester of Lev), 170.36 (amide), 139.89, 139.07, 138.98, 138.89, 138.83, 138.65, 138.62, 138.58, 138.56, 138.36, 138.27, 138.05, 138.02, 137.74, 128.64, 128.60, 128.58, 128.46, 128.43, 128.40, 128.38, 128.33, 128.32, 128.30, 128.27, 128.22, 128.18, 128.15, 128.09, 128.05, 128.02, 127.96, 127.86, 127.78, 127.73, 127.68, 127.66, 127.62, 127.57, 127.53, 127.50, 127.47, 127.45, 127.28, 127.26, 127.16, 127.10, 126.97, 100.64 (GalNAc-1), 99.51, 98.86, 98.71, 96.74 (GlcNH₂-1), 81.98, 81.55, 81.16, 80.44, 79.82, 79.27, 77.36, 75.93, 75.74, 75.59, 75.46, 75.34, 75.18, 74.88, 74.59, 74.41, 74.36, 73.93, 73.88, 73.63, 73.28, 73.17, 72.71, 72.49, 72.37, 72.26, 72.10, 71.61, 71.52, 71.13, 70.80, 70.79, 70.71, 70.68, 70.62, 69.96, 69.81, 69.01, 68.73, 66.76, 63.92, 63.68, 62.95, 62.86, 45.59 (CH₂ of TEA), 37.91 (CH₂ of Lev), 34.40, 34.19 (2 x O-COCH₂-CH₂), 32.05, 29.84, 29.81, 29.79, 29.67, 29.65, 29.48, 29.45, 29.28, 28.07 (CH₂ of Lev), 25.02, 24.99 (2 x O-COCH₂-CH₂), 23.22 (Me of NHAc), 22.81, 18.23, 18.18 (2x TIPS), 14.23, 14.22 (2 x Me of lipid chain), 12.18 (TIPS), 8.57 (Me of TEA); ³¹P NMR (243 MHz, CDCl₃) δ -1.42; ESI-MS for C₂₀₃H₂₆₁N₄O₃₉PSi: m/z [M-H+3Na]²⁺ cald 1675.8204, obsd 1675.8225.

### Example 20

### Triethylammonium 2,3,4-tri-O-benzy)-α-D-mannopyranosy)-(1→2)-3,4,6-tri-O-benzyl-α-D-mannopyranosyl-(1→6)-4-O-(3,4,6-tri-O-benzyl-2-deoxy-2-acetamido-β-D-galactopyranosyl)-3-O-benzyl-2-O-levulinyl-α-D-mannopyranosyl-(1→4)-2-azido-3,6-di-O-benzyl-2-deoxy-α-D-glucopyranosyl-(1→6)-2,3,4,5-tetra-O-benzyl-1-O-(1,2-di-O-octadecanoy)-sn-glyceryl-phosphonato)-D-myo-inositol (19)

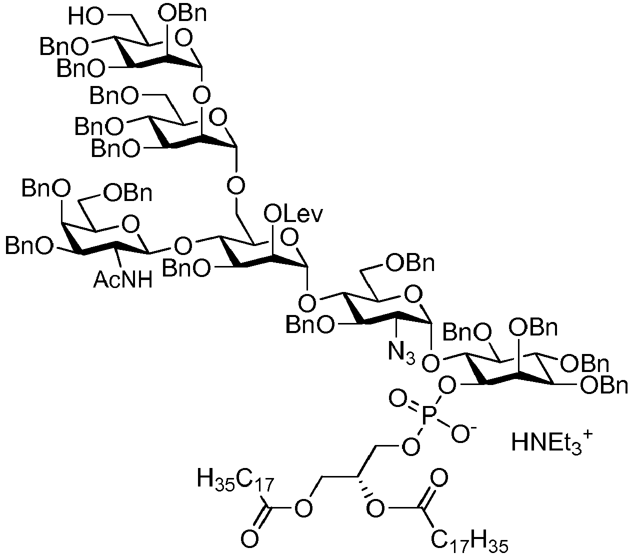

The lipidated pseudohexasaccharide **19a** of Example 19 (56 mg, 0.016 mmol) was dissolved in MeCN (3 mL). Water (11.4 µL, 0.633 mmol) and Sc(OTf)₃ (23.4 mg, 0.047 mmol) were added and the reaction mixture was stirred at 50 °C for 5 h before it was quenched with TEA (50 µL) and evaporated to dryness. The residue was purified using flash column chromatography (CHCl₃/MeOH from 100:0 -> 97:3). The fractions containing the desired product were pooled and washed with TEA/CO₂-Buffer (2 x 15 mL), dried over Na₂SO₄ and concentrated to yield pseudohexasaccharide **19** (38 mg, 0.011 mmol, 71 % yield) as white foam: [α]²⁰_{D} = + 37.1 (c = 1.00, CHCl₃); ATR-FTIR (cm⁻¹): 3298, 3089, 3063, 3031, 2924, 2854, 2108, 1740, 1674, 1454, 1362, 1211, 1096, 1049, 1028; ¹H NMR (400 MHz, CDCl₃) δ 12.25 (s, 1 H, **H**NEt₃⁺), 7.33 (d, J = 7.1 Hz, 2H), 7.31 - 6.99 (m, 78H), 6.00 (d, J = 8.3 Hz, 1 H, NH), 5.81 (d, J = 3.5 Hz, 1 H, GlcNH₂-1), 5.30 - 5.19 (m, 2H), 5.15 (dq, J = 5.9, 3.3 Hz, 1 H, CH of glycerol), 4.94 (dd, J = 6.6, 5.2 Hz, 2H), 4.88 - 4.63 (m, 11 H), 4.61 - 4.14 (m, 20H), 4.11 -3.34 (m, 31 H), 3.16 (q, J = 9.3 Hz, 1H), 3.05 (dd, J = 10.1, 3.6 Hz, 1 H), 2.87 (dd, J = 13.4, 8.8 Hz, 6H, CH₂ of TEA), 2.29 - 2.09 (m, 8H, CH₂ of Lev; 2x O-COC**H**₂-CH₂), 1.85 (s, 3H, Me of Lev), 1.64 (s, 3H, Me of NHAc), 1.51 - 1.42 (m, 4H, 2x O-COCH₂-C**H**₂), 1.26 - 1.10 (m, 65H, Me of TEA; CH₂ of lipid), 0.81 (t, J = 6.8 Hz, 6H, Me of lipid); ¹³C NMR (101 MHz, CDCl₃) δ 206.21 (ketone), 173.49, 173.12, 171.74, 170.73, 139.89, 139.03, 138.86, 138.70, 138.63, 138.53, 138.49, 138.44, 138.37, 138.21, 138.10, 138.02, 130.13, 129.86, 128.67, 128.56, 128.50, 128.47, 128.41, 128.37, 128.35, 128.34, 128.31, 128.28, 128.14, 128.12, 128.04, 128.02, 127.99, 127.84, 127.79, 127.76, 127.69, 127.67, 127.63, 127.61, 127.58, 127.54, 127.50, 127.46, 127.35, 127.28, 127.21, 127.12, 127.05, 100.79, 99.89, 99.38, 98.99, 96.60, 81.91, 81.60, 81.15, 80.04, 79.56, 79.12, 77.36, 76.03, 75.91, 75.72, 75.41, 75.28, 75.17, 75.12, 74.97, 74.84, 74.56, 74.34, 74.24, 74.12, 73.96, 73.54, 73.50, 73.45, 73.10, 73.06, 72.45, 72.32, 72.25, 72.18, 71.72, 71.34, 70.71, 70.64, 70.00, 69.70, 69.47, 68.87, 68.71, 67.21, 63.85, 63.36, 62.90, 62.41, 54.24, 45.64, 37.90, 34.41, 34.20, 32.06, 29.85, 29.82, 29.80, 29.72, 29.68, 29.66, 29.50, 29.46, 29.38, 29.34, 29.29, 28.02, 27.35, 27.30, 25.64, 25.03, 25.00, 23.45, 22.82, 14.26, 8.61; ³¹P NMR (243 MHz, CDCl₃) δ -1.58; ESI-MS for C₁₉₄H₂₄₁N₄O₃₉P: m/z [M-H+3Na]²⁺ cald 1753.8871, obsd 1753.8871.

### Example 21

### Bistriethylammonium (2,3,4-tri-O-benzyl-6-O-(2-(N-benzyloxycarbonyl)aminoethyl-phosphonato)-α-D-mannopyranosyl-(1→2)-3,4,6-tri-O-benzyl-α-D-mannopyranosyl-(1→6)-4-O-(3,4,6-tri-O-benzy)-2-deoxy-2-acetamido-β-D-galactopyranosyl)-3-O-benzyl-α-D-mannopyranosyl-(1→4)-2-azido-3,6-di-O-benzyl-2-deoxy-α-D-glucopyranosyl-(1→6)-2,3,4,5-tetra-O-benzyl-1-O-(1,2-di-O-octadecanoyl-sn-glycerylphosphonato)-D-myo-inositol (20)

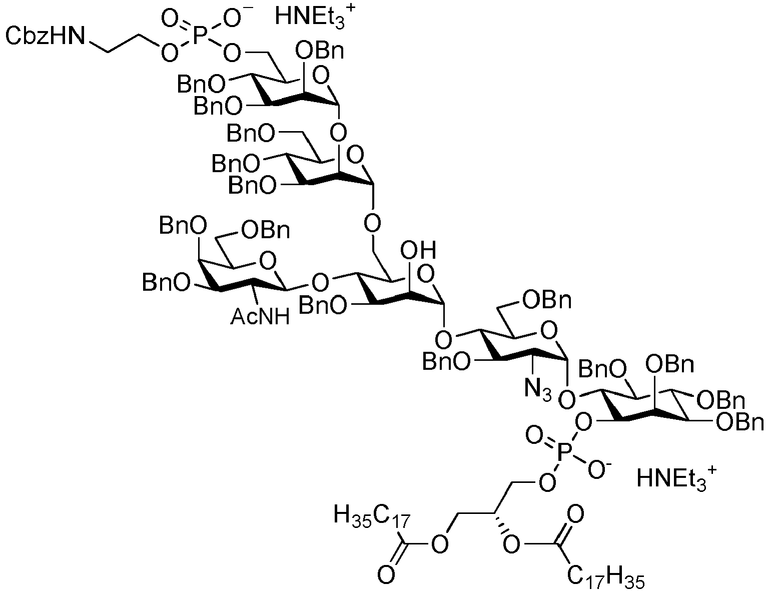

The lipidated pseudohexasaccharide **19** of Example 20 (28 mg, 8.3 µmol) and triethylammonium 2-[(N-benzyloxycarbonyl)amino]ethyl H-phosphonate (13.4 mg, 37 µmol) were evaporated with anhydrous pyridine (3 x 2 mL) and placed under high vacuum for 30 min. The residue was dissolved in anhydrous pyridine (2 mL) and PivCl (7.6 µL, 62 µmol) was added. The reaction mixture was stirred for 2 h at room temperature before water (50 µL) and iodine (11.5 mg, 45µmol) were added. The reaction mixture was stirred for 1 h before acetic acid (95 µL, 1.654mmol) and hydrazine monohydrate (65 wt%, 40 µL, 0.827 mmol) were added. After 12 h the solvents were evaporated and the residue was evaporated with toluene (3 x 2 mL). The crude product was purified using flash column chromatography (CHCl₃/MeOH from 100:0 -> 90:10). The fractions containing the desired product were pooled and washed with TEA/CO₂-Buffer (2 x 15mL), dried over Na₂SO₄ and concentrated to yield bisphosphorylated pseudohexasaccharide **20** (24 mg, 6.6 µmol, 80% yield) as colorless oil: R_{f} (SiO₂, CHCl₃/MeOH 90:10) = 0.38; [α]²⁰_{D}: + 28.2 (c = 1.00, CHCl₃); ATR-FTIR (cm⁻¹): 3254, 3030, 2924, 2853, 2107, 1737, 1672, 1497, 1454, 1362, 1215, 1052, 1028; ¹H NMR (600 MHz, CDCl₃) δ 12.17 (s, 2H, **H**NEt₃), 7.46 - 6.94 (m, 85H), 6.28 (s, 1 H, N**H**), 5.84 (d, J = 3.4 Hz, 1 H, GlcNH₂-1), 5.29 - 5.13 (m, 2H, C**H** of glycerole), 4.99 - 4.16 (m, 41 H), 4.15 - 4.00 (m, 5H), 3.99 - 3.39 (m, 32H), 3.28 - 3.22 (m, 1 H), 3.14 (dd, J = 9.2, 4.6 Hz, 1 H), 3.04 (dd, J = 10.2, 3.4 Hz, 1 H, GlcNH₂-2), 2.80 (dd, J = 13.5, 6.5 Hz, 12H, CH₂ of TEA), 2.21 - 2.11 (m, 4H), 1.82 (s, 3H, Me of NHAc), 1.54 - 1.43 (m, 4H), 1.30 - 1.15 (m, 56H), 1.10 (t, J = 7.2 Hz, 18H, Me of TEA), 0.81 (t, J = 7.0 Hz, 6H, Me of lipid); ¹³C NMR (101 MHz, CDCl₃) δ 173.48, 173.11 (2x fatty acid ester), 170.88 (amide), 156.61 (Cbz), 139.90, 139.22, 139.02, 138.93, 138.87, 138.69, 138.56, 138.46, 138.30, 138.22, 137.95, 137.71, 137.12, 128.68, 128.50, 128.41, 128.37, 128.33, 128.29, 128.26, 128.24, 128.20, 128.09, 127.98, 127.91, 127.84, 127.81, 127.76, 127.68, 127.66, 127.61, 127.55, 127.42, 127.31, 127.11, 126.94, 102.02, 100.88, 99.89, 98.46, 96.49 (GlcNH₂-1), 81.89, 81.81, 81.08, 80.28, 80.11, 79.75, 79.72, 79.65, 79.60, 79.54, 78.20, 78.16, 76.31, 75.82, 75.63, 75.25, 75.14, 74.88, 74.78, 74.54, 74.40, 73.52, 73.40, 73.26, 73.04, 72.69, 72.42, 72.30, 72.24, 72.04, 71.98, 71.73, 70.75, 70.71, 70.07, 69.76, 69.42, 68.96, 66.38, 65.23, 64.23, 63.88, 63.43 (GlcNH₂-2), 61.85, 45.61 (CH₂ of TEA), 34.41, 34.20 (2x O-CO-**C**H₂-CH₂), 32.06, 29.85, 29.80, 29.67, 29.50, 29.30, 25.04, 25.00 (2x O-CO-CH₂-**C**H₂), 23.51 (Me of AcNH), 22.83, 14.26 (Me of lipid), 8.58 (Me of TEA); ³¹P NMR (243 MHz, CDCl₃) δ 0.16, -1.69; ESI-MS for C₁₉₉H₂₄₇N₅O₄₂P₂: m/z [M-2H+3Na+K]²⁺ cald 1774.3026, obsd 1774.2933.

### Example 22

### 6-O-Aminoethylphosphonato-α-D-mannopyranosyl-(1→2)-α-D-mannopyranosyl-(1→6)-4-O-(2-deoxy-2-acetamido-β-D-galactopyranosyl)-α-D-mannopyranosyl-(1→4)-2-amino-2-deoxy-α-D-glucopyranosyl-(1→6)-1-O-(1,2-di-O-octadecanoyl-sn-glycerylphosphonato)-D-myo-inositol (GPI B)^{[4]}

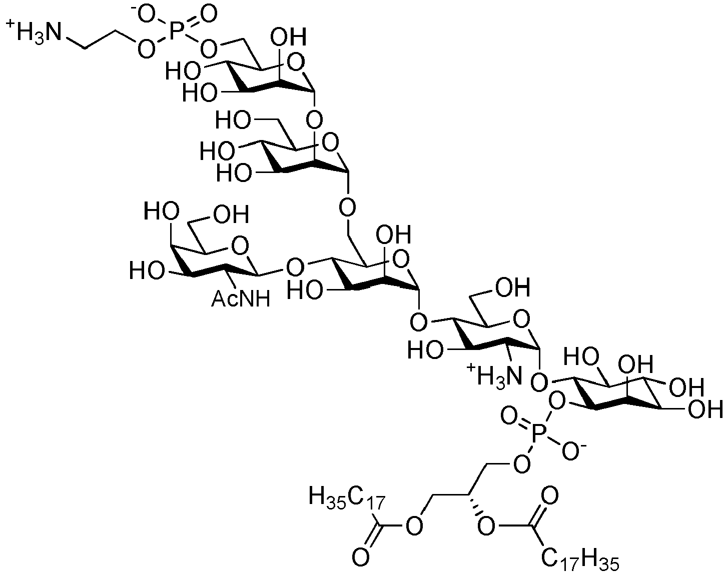

The bisphosphorylated pseudohexasaccharide **20** of Example 21 (7.0 mg, 1.9 µmol) was dissolved in CHCl₃ (2mL) and Amberlite IR120 H (30 mg) was added. The slurry was stirred for 1 h, before it was filtered and evaporated to dryness. Afterwards the residue was dissolved in CHCl₃/MeOH/H₂O (4 mL, 9:7:2). Pd/C (10wt%, 20.4 mg, 19 µmol) and HOAc (10µL) were added. Through this slurry was bubbled H₂ for 15 min, before the reaction mixture was stirred for 5 d under a hydrogen atmosphere. The solution was filtered through a syringe filter and evaporated to dryness to yield GPI **B** (3.1 mg, 1.7 µmol, 89 % yield) as white solid. Spectral data was in agreement with previously reported data.

### Example 23 Conjugation of compounds of formula (I) on a carrier

For mild alkaline methanolysis (saponification), the synthetic GPI **A** and **B** were dried and dissolved in 250 µL of methanol (final concentration 1mM) and sonicated for 5 min. Sodium hydroxide was then added to reach a final concentration of 100 mM, and the reaction mixture was incubated for 2 h at 37°C, after which 2 µL of concentrated acetic acid was added. The reaction mixture was dried and partitioned between water and water/saturated *n-*butanol.

### Example 24 Diagnosis of toxoplasmosis testing serum of patients

The glycans 21 and 22 obtained (concentration = 1mM) were dissolved in sodium phosphate buffer (50 mM, pH 8.5) and robotically printed using a piezoelectric spotting device (S3, Scienion, Berlin, Germany) onto NHS-activated CodeLink slides (Amersham Biosciences), in 50% relative humidity, at 23 °C to give average spot sizes of 200 µm. Immobilization of the structures yielded a signal to noise ratio of z 10. Slides were stored in an anhydrous environment. Prior to the experiment, the slides were washed three times with water and the remaining succinimidyl groups were quenched by incubating the slides in 100 mM ethanolamine in 50 mM PBS (pH 9) for 1 h at 50 °C. The slides were then rinsed three times with water and dried by centrifugation.

### Testing serum of patients using the slides obtained

The slides obtained were blocked using 1% BSA solved in PBS, pH 7.4 and rinsed three times with water (3x10 mL). Subsequently the slides were incubated for 1 hour with serum probes which were diluted 1:100 in PBS and rinsed three times with PBSr (3x10 mL). Afterwards the slides were incubated for 1 hour using an antihuman IgG/IgM antibody marked with ALEXA-Fluor®647 (Invitrogen) diluted 1:1000 in PBS (0.5% BSA and 0.05% Tween-20, pH 7.4) and rinsed with PBS (3x10 mL). The slides were then washed with water for 3 s, dried by centrifugation and analyzed using a fluorescence microarray scanner (Genepix ® 4300A, Molecular Devices). All signals with a signal-to-noise ratio> 3 were scored as positive and classified as latent toxoplasmosis (Figure 5 shows an example for one patient).

### Results:

In total, 94 serum samples were analyzed and the consistency to the conventional analysis, which consists of a commercial toxoplasmosis kit (ELISA Vidas bioMérieux) and a histological immunofluorescence analysis, was 78-83% for a latent toxoplasmosis.

The test therefore has the following statistical values:Sensitivity = 82%
Specificity = 85%
Positive prediction value = 90%
Negative prediction Value = 73%

These values are remarkable for a serological test and in combination with available ELISA tests (Garry et al., 2005, Infectious Diseases in Obstetrics and Gynecology 13, 151-153) lead to a clear distinction between latent and acute toxoplasmosis.

## Claims

1. Diagnostic device comprising at least one compound of general formula (I) 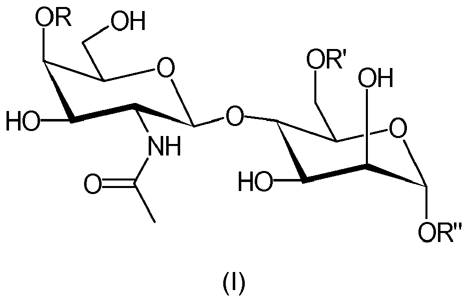 wherein
R represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -Ph, -CH₂-Ph, or 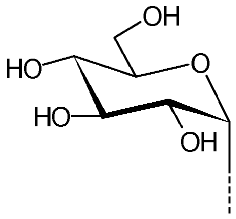
R' represents -H or 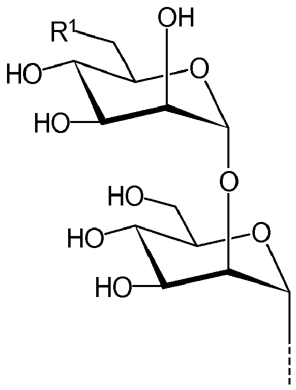
R¹ represents -OH or -OP(O)(OH)-O-X-NH₂;
X represents -CH₂-, -C₂H₄-, -C₃H₆-, -C₄H₈-, -C₅H₁₀-, or -C₆H₁₂-;
R" represents -L-R², -(C₂H₄O)ᵣ-CH₂-R², -(C₂H₄O)ᵣ-C₂H₄-R² or 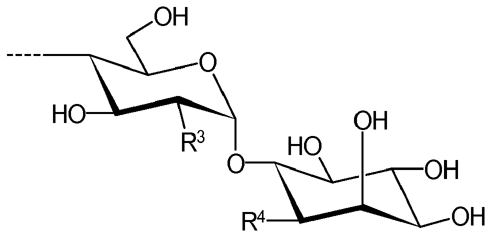
R² and R³ represent independently of each other -H, -OH, -NH₂, -SH, -N₃, -NHCOCH₃, -NHCOCH₂CH₃, or -NHCOCH₂CH₂CH₃;
R⁴ represents -OH, -SO²(R⁵), -OSO₂(R⁵), -OSO₂(OR⁵), or -OP(O)(OR⁵)(OR⁶);
R⁵ and R⁶ represent independently of each other -H, -L-OH, -(C₂H₄O)ᵣ-CH₂-OH or -(C₂H₄O)ᵣ-C₂H₄-OH;
L represents a linker;
and r is an integer from 1 to 40,
for diagnosis of toxoplasmosis and wherein the at least one compound of general formula (I) is immobilized on a carrier by covalent bonding.

2. Diagnostic device according to claim 1 for diagnosis of acute toxoplasmosis.

3. Diagnostic device according to claims 1 or 2 wherein the carrier is selected from the group comprising a glass slide, a microtitre plate, microspheres, beads.

4. Diagnostic device according to any one of the claims 1 - 3 wherein the at least one compound of general formula (I) is covalently bound using a linking molecule.

5. A compound of general formula (I) 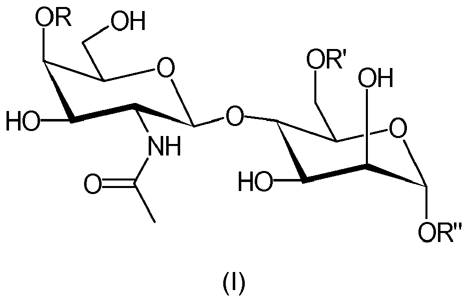 wherein
R represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -Ph, -CH₂-Ph, or 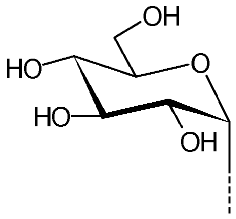
R' represents -H or 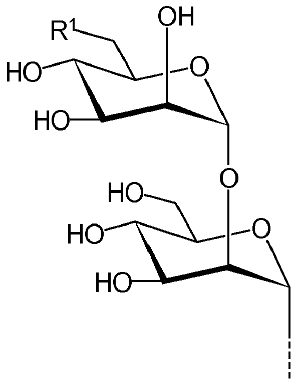
R¹ represents -OH or -OP(O)(OH)-O-C₂H₄-NH₂;
R" represents -L-R², -(C₂H₄0)ᵣ-CH₂-R², -(C₂H₄O)ᵣ-C₂H₄-R² or 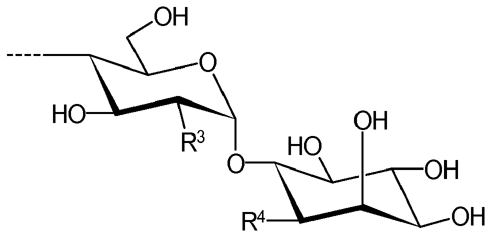
R² and R³ represent independently of each other -H, -OH, -NH₂, -SH, -NHCOCH₃, -NHCOCH₂CH₃, or -NHCOCH₂CH₂CH₃;
R⁴ represents -OH, -SO₂(R⁵), -OSO₂(R⁵), -OSO₂(OR⁵), or -OP(O)(OR⁵)(OR⁶);
R⁵ and R⁶ represent independently of each other -H, -L-OH, -(C₂H₄O)ᵣ-CH₂-OH or -(C₂H₄O)ᵣ-C₂H₄-OH;
L represents a linker;
and r is an integer from 1 to 40.

6. Compound according to claim 5 covalently linked to an adjuvant.

7. Compound according to claim 6 wherein the adjuvant is selected from the group comprising a diphtheria toxoid, a mutated diphtheria toxoid, a modified diphtheria toxoid, a modified and mutated diphtheria toxoid, or a tetanus toxoid.

8. Compound according to claim 5 immobilized on a carrier by covalent bonding.

9. Use of at least one compound according to claim 5 unbound or immobilized on a carrier for diagnosis of toxoplasmosis.

10. Use according to claim 9 wherein the diagnosis of toxoplasmosis is based on the detection of the presence of antibodies specific for at least one compound of general formula (I) as defined in claim 5.

11. Use according to claim 9 or 10 for diagnosis of acute toxoplasmosis.

12. Use of the diagnostic device according to any one of claims 1 - 4 for diagnosis of toxoplasmosis.

13. Compounds according to claim 5 or 6 for vaccination against toxoplasmosis.

14. A kit including at least the following components:
A) A compound of general formula (I) immobilized on a carrier by covalent bonding;
B) at least one antibody:
and
C) a standard solution.
